# EUROPÄISCHE PATENTSCHRIFT

(11) **EP 2 836 592 B1**
(45) Veröffentlichungstag und Bekanntmachung des Hinweises auf die Patenterteilung: **16.05.2018**
(21) Anmeldenummer: 13709422.3
(22) Anmeldetag: 13.03.2013
(51) Int. Cl.: C12N 9/12

(54) **ACETYLTRANSFERASE VON WICKERHAMOMYCES CIFERRII**
ACETYL TRANSFERASE FROM WICKERHAMOMYCES CIFERRII
ACÉTYLTRANSFÉRASE DE WICKERHAMOMYCES CIFERRII

(30) Priorität: 11.04.2012 DE 102012007491
(43) Veröffentlichungstag der Anmeldung: 18.02.2015
(73) Patentinhaber: Evonik Degussa GmbH, 45128 Essen (DE)
(72) Erfinder: SCHAFFER, Steffen, 45699 Herten (DE); FARWICK, Mike, 45138 Essen (DE); ANDREA, Heiko, 45768 Marl (DE); KÖHLER, Tim, 46284 Dorsten (DE); WOLFF, Daniel, 44805 Bochum (DE); TER VELD, Frank, 42287 Wuppertal (DE); POETSCH, Ansgar, 44797 Bochun (DE); BOLES, Eckhard, 64293 Darmstadt (DE); SCHORSCH, Christoph, 60316 Frankfurt am Main (DE)
(86) Internationale Anmeldenummer: PCT/EP2013/055092
(87) Internationale Veröffentlichungsnummer: WO 2013/152913

(56) Entgegenhaltungen:
- KRISTOF DE SCHUTTER ET AL: "Genome sequence of the recombinant protein production host Pichia pastoris", NATURE BIOTECHNOLOGY, NATURE PUBLISHING GROUP, NEW YORK, NY, US, Bd. 27, Nr. 6, 1. Juni 2009 (2009-06-01), Seiten 561-566, XP008156426, ISSN: 1087-0156, DOI: 10.1038/NBT.1544 -& DATABASE UniProt [Online] 7. Juli 2009 (2009-07-07), "SubName: Full=N-acetyltransferase, confers resistance to the sphingolipid biosynthesis inhibitor myriocin (ISP-1);", XP002696842, gefunden im EBI accession no. UNIPROT:C4R7I7 Database accession no. C4R7I7
- ANDREAS KÜBERL ET AL: "High-quality genome sequence ofCBS7435", JOURNAL OF BIOTECHNOLOGY, ELSEVIER SCIENCE PUBLISHERS, AMSTERDAM, NL, Bd. 154, Nr. 4, 26. April 2011 (2011-04-26), Seiten 312-320, XP028240769, ISSN: 0168-1656, DOI: 10.1016/J.JBIOTEC.2011.04.014 [gefunden am 2011-05-06]
- CHRISTOPH SCHORSCH ET AL: "High-level production of tetraacetyl phytosphingosine (TAPS) by combined genetic engineering of sphingoid base biosynthesis and L-serine availability in the non-conventional yeast Pichia ciferrii", METABOLIC ENGINEERING, Bd. 14, Nr. 2, 1. März 2012 (2012-03-01), Seiten 172-184, XP055038100, ISSN: 1096-7176, DOI: 10.1016/j.ymben.2011.12.002
- DANIEL BÖRGEL ET AL: "Metabolic engineering of the non-conventional yeast Pichia ciferrii for production of rare sphingoid bases", METABOLIC ENGINEERING, Bd. 14, Nr. 4, 18. März 2012 (2012-03-18), Seiten 412-426, XP055037974, ISSN: 1096-7176, DOI: 10.1016/j.ymben.2012.03.003
- J. SCHNEIDER ET AL: "Draft Genome Sequence of Wickerhamomyces ciferrii NRRL Y-1031 F-60-10", EUKARYOTIC CELL, Bd. 11, Nr. 12, 1. Dezember 2012 (2012-12-01), Seiten 1582-1583, XP055062220, ISSN: 1535-9778, DOI: 10.1128/EC.00258-12 -& DATABASE UniProt [Online] 28. November 2012 (2012-11-28), "SubName: Full=N-acetyltransferase SLI1; EC=2.3.1.-;", XP002696843, gefunden im EBI accession no. UNIPROT:K0KN92 Database accession no. K0KN92
- BARENHOLZ Y ET AL: "Acetylation of sphingosine bases and long-chain amines by cell-free preparations of Hansenula ciferri", BIOCHEMICAL AND BIOPHYSICAL RESEARCH COMMUNICATIONS, ACADEMIC PRESS INC. ORLANDO, FL, US, vol. 35, no. 5, 6 June 1969 (1969-06-06), pages 676-680, XP024844957, ISSN: 0006-291X, DOI: 10.1016/0006-291X(69)90458-6 [retrieved on 1969-06-06]
- BÖGEL D.: "UNTERSUCHUNGEN ZUR SPHINGOLIPID-BIOSYNTHESE IN DER HEFE PICHIA CIFERRII", JOHANN WOLFGANG GOETHE UNIVERSITÄT, 2007, pages 28-63,
- DATABASE EMBL [Online] 12 Februar 2012 'Wickerhamomyces anomalus putative alcohol acetyltransferase (WAN_11037) gene, complete cds.' Retrieved from EBI, accession no. EM_STD:JN701428 Database accession no. JN701428

## Beschreibung

### Gebiet der Erfindung

Gegenstand der Erfindung sind neue Enzyme, die acetylierte Sphingoidbasen bereitstellen.

### Stand der Technik

*Pichia ciferrii* wird bereits seit Anfang der 60er Jahre zur Herstellung von Sphingoidbasen und Sphingolipiden verwendet.

Die Ausbeuten der Wildtypstämme an Sphingoidbasen und Sphingolipiden ist stets verbesserungswürdig.

Sphingoidbasen, insbesondere Phytosphingosin, Sphingosin und Sphinganin finden vielfältigen Einsatz als kosmetische Wirkstoffe zum Schutz und zur Pflege der Haut.

Sie werden entweder direkt oder nach chemischer Umsetzung in hautidentische Ceramide in kosmetische Pflegeprodukte eingearbeitet.

Die nicht-konventionelle Hefe *Pichia ciferrii* zeichnet sich dadurch aus, dass sie relativ hohe Mengen an acetylierten Sphingoidbasen, hauptsächlich Tetraacetylphytosphingosin (TAPS) und Triacetylsphinganin (TriASa), ins Kultivierungsmedium sekretiert. Das gebildete TAPS lässt sich mittels Extraktion aus der Kulturbrühe extrahieren und wird nachfolgend chemisch in freies Phytosphingosin, sowie in verschiedene Ceramide, umgesetzt.

Eine effiziente Acetylierung ist Grundvoraussetzung für den Transport der Sphingoidbasen ins Kulturmedium: Enzymatische Tests mit Mikrosomenfraktionen zeigten, dass diejenigen Stämme mit hoher Produktivität acetylierter Sphingoidbasen (Hoch-Produzenten) eine deutlich gesteigerte spezifische Acetyltransferase Aktivität aufwiesen als die Niedrig-Produzenten (Barenholz et al., 1971; Barenhoz et al., 1973).

Damit konnte diese enzymatische Aktivität als eine der Hauptursachen für die effiziente Produktion acetylierter Sphingoidbasen durch verschiedene *Pichia ciferrii-*Stämme festgemacht werden.

Alle Versuche zur Aufreinigung, Charakterisierung und Identifizierung solcher Enzyme schlugen bisher fehl, so dass weder die Proteine, noch die entsprechenden Gene bekannt sind.

Aufgabe der Erfindung war es, Enzyme und ihre kodierenden Sequenzen bereit zustellen, welche Sphingoidbasen zu acetylieren vermögen.

### Beschreibung der Erfindung

Überraschenderweise wurde gefunden, dass die im Folgenden beschriebenen Enzyme die der Erfindung gestellte Aufgabe zu lösen vermögen.

Gegenstand der vorliegenden Erfindung sind daher isolierte, für Acetyltransferasen kodierende Nukleinsäuren wie in den Ansprüchen beschrieben.
Ein weiterer Gegenstand der Erfindung sind rekombinante Zellen, die eine geänderte Aktivität der erfindungsgemäßen Enzyme aufweisen.

Die mit der vorliegenden Erfindung beschriebenen Acetyltransferasen, bzw. die sie kodierenden DNA-Sequenzen, bieten eine Reihe von Vorteilen. Sie lassen sich dazu nutzen, definierte Substrate (Sphingoidbasen) biotechnologisch und hochspezifisch an verschiedenen funktionellen Gruppen (Hydroxy- und Aminogruppen) zu acetylieren. Im Vergleich zu einem chemischen Acetylierungsprozess werden dabei weniger Nebenprodukte generiert, wodurch der Ausbeuteverluste und aufwendige Reinigungsschritte minimiert werden können. Vor allem für Anwendungszwecke, die eine hohe Produktreinheit erfordern, bietet die Erfindung somit ein hohes Potential. Besonders hohe Produktreinheiten sind u.a. in den Sparten Kosmetik, Nahrungs- und Genußmittel, Pharmazeutik erforderlich, so daß hier die Erfindung besonders hohes Potential hat. Die biotechnologische Herstellung acetylierter Sphingoidbasen kann mit der vorliegenden Erfindung grundsätzlich auf zwei verschiedene Arten realisiert werden: Zum einen kann die Herstellung über den Ansatz der Biokatalyse erfolgen, indem ausgewählte Sphingoidbasen in einem geeigneten Reaktor unter Zugabe der Acetyltransferase(n) enzymatisch acetyliert werden. Zum anderen lassen sich die Gene der Acetyltransferasen nutzen, um mittels gentechnischer Methoden (*Metabolic Engineering*) rekombinante Mikrobenstämme zu generieren, welche direkt dazu befähigt sind, in einem fermentativen Prozess acetylierte Sphingoidbasen aus einfachen C- und N-Quellen zu synthetisieren. Im Vergleich zu einem chemischen Prozess ist der fermentative kostengünstiger und ökologisch nachhaltiger. Desweiteren ist er stereospezifisch, was bei einer chemischen Vollsynthese nicht gewährleistet ist. Durch Verwendung nur einer der in dieser Erfindung beschriebenen Acetyltransferasen bzw. ihrer Gene lassen sich gezielt unvollständig acetylierte Sphingoidbasen erzeugen.

Diese haben eventuell besondere biologische Wirkungen und können somit für besondere Applikationen verwendet werden, z.B. als kosmetische oder pharmazeutische Wirksoffe oder deren Vorstufen. Unvollständig acetylierte Sphingoidbasen sind chemisch nur äusserst aufwendig herzustellen, wodurch für den biotechnologischen Ansatz ein erheblicher Kostenvorteil entsteht.

Alle angegebenen Prozent (%) sind, wenn nicht anders angegeben, Massenprozent.

Einen Beitrag zur Lösung der Aufgabe leistet eine isolierte Nukleinsäure, welche eine Sequenz ausgewählt aus den Gruppen [A1 bis D1 und G1] aufweist
A1) eine Sequenz gemäß Seq ID Nr. 1, wobei diese Sequenz für ein Protein kodiert, welches in der Lage ist,
   Phytosphingosin durch Übertragung der Acetylreste aus drei Molekülen Acetyl Coenzym A zu Triacetylphytosphingosin umzusetzen,
B1) eine intronfreie Sequenz, die von einer Sequenz nach A1) abgeleitet ist und die das gleiche Protein oder Peptid kodiert wie die Sequenz nach Seq ID Nr. 1,
C1) eine Sequenz, die ein Protein oder Peptid kodiert, das die Aminosäuresequenz gemäß Seq ID Nr. 2 umfasst und das in der Lage ist,
   Phytosphingosin durch Übertragung der Acetylreste aus drei Molekülen Acetyl Coenzym A zu Triacetylphytosphingosin umzusetzen,
D1) eine Sequenz, die mit einer Sequenz nach einer der Gruppen A1) bis C1), besonders bevorzugt nach Gruppe A1), zu mindestens 70%, besonders bevorzugt zu mindestens 90%, darüber hinaus bevorzugt zu mindestens 95% und am meisten bevorzugt zu mindestens 99% identisch ist, wobei diese Sequenz für ein Protein oder Peptid kodiert, welches in der Lage ist,
   Phytosphingosin durch Übertragung der Acetylreste aus drei Molekülen Acetyl Coenzym A zu Triacetylphytosphingosin umzusetzen,
G1) eine komplementäre Sequenz zu einer Sequenz nach einer der Gruppen A1) bis D1), besonders bevorzugt nach Gruppe A1).

Einen weiteren Beitrag zur Lösung der Aufgabe leistet eine isolierte Nukleinsäure, welche eine Sequenz ausgewählt aus den Gruppen [A2 bis D2 und G2] aufweist
A2) eine Sequenz gemäß Seq ID Nr. 3, wobei diese Sequenz für ein Protein kodiert, welches in der Lage ist,
   Triacetylphytosphingosin durch Übertragung des Acetylrestes aus einem Molekül Acetyl Coenzym A zu Tetraacetylphytosphingosin umzusetzen,
B2) eine intronfreie Sequenz, die von einer Sequenz nach A2) abgeleitet ist und die das gleiche Protein oder Peptid kodiert wie die Sequenz nach Seq ID Nr. 3,
C2) eine Sequenz, die ein Protein oder Peptid kodiert, das die Aminosäuresequenz gemäß Seq ID Nr. 4 umfasst, und welches in der Lage ist,
   Triacetylphytosphingosin durch Übertragung des Acetylrestes aus einem Molekül Acetyl Coenzym A zu Tetraacetylphytosphingosin umzusetzen,
D2) eine Sequenz, die mit einer Sequenz nach einer der Gruppen A2) bis C2), besonders bevorzugt nach Gruppe A2), zu mindestens 70%, besonders bevorzugt zu mindestens 90%, darüber hinaus bevorzugt zu mindestens 95% und am meisten bevorzugt zu mindestens 99% identisch ist, wobei diese Sequenz für ein Protein oder Peptid kodiert, welches in der Lage ist,
   Triacetylphytosphingosin durch Übertragung des Acetylrestes aus einem Molekül Acetyl Coenzym A zu Tetraacetylphytosphingosin umzusetzen,
G2) eine komplementäre Sequenz zu einer Sequenz nach einer der Gruppen A2) bis D2), besonders bevorzugt nach Gruppe A2),

Die "Nukleotid-Identität" oder "Aminosäure-Identität" wird hier mit Hilfe bekannter Verfahren bestimmt. Generell werden besondere Computerprogramme mit Algorithmen unter Berücksichtigung spezieller Erfordernisse verwendet.

Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG-Programmpaket, einschließlich

GAP (Deveroy, J. et al., Nucleic Acid Research 12 (1984), Seite 387, Genetics Computer Group University of Wisconsin, Medicine (Wi), und BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. *et al*., vorstehend).

Der bekannte Smith-Waterman Algorithmus kann ebenso zur Bestimmung der Nukleotid-Identität verwendet werden.

Bevorzugte Parameter für die Bestimmung der "Nukleotid-Identität" sind bei Verwendung des BLASTN-Programms (Altschul, S. et al., Journal of Molecular Biology 215 (1990), Seiten 403-410:

| | |
|---|---|
| Expect Threshold: | 10 |
| Word size: | 28 |
| Match Score: | 1 |
| Mismatch Score: | -2 |
| Gap costs: | Linear |

Die vorstehenden Parameter sind die default-Parameter im Nukleotidsequenzvergleich.

Das GAP-Programm ist ebenso zur Verwendung mit den vorstehenden Parametern geeignet.

Bevorzugte Parameter für die Bestimmung der "Aminosäure-Identität" sind bei Verwendung des BLASTP-Programms (Altschul, S. et al., Journal of Molecular Biology 215 (1990), Seiten 403-410:

| | |
|---|---|
| Expect Threshold: | 10 |
| Word size: | 3 |
| Matrix: | BLOSUM62 |
| Gap costs: | Existence: 11; Extension: 1 |
| Compositional adjustments: | Conditional compositional score matrix adjustment |

Die vorstehenden Parameter sind die default-Parameter im Aminosäuresequenzvergleich.
Das GAP-Programm ist ebenso zur Verwendung mit den vorstehenden Parametern geeignet.

Eine Identität von 60% gemäß dem vorstehenden Algorithmus bedeutet im Zusammenhang mit der vorliegenden Erfindung 60% Identität. Das gleiche gilt für höhere Identitäten.

Das Merkmal "Sequenz, die mit dem Gegenstrang einer Sequenz hybridisiert oder unter Berücksichtigung der Degeneration des genetischen Codes hybridisieren würde" weist auf eine Sequenz hin, die unter vorzugsweise stringenten Bedingungen mit dem Gegenstrang einer Bezugssequenz hybridisiert oder unter Berücksichtigung der Degeneration des genetischen Codes hybridisieren würde. Beispielsweise können die Hybridisierungen bei 68°C in 2 x SSC oder nach dem Protokoll des Digoxigenin-Labelling-Kits der Firma Boehringer (Mannheim) durchgeführt werden. Bevorzugte Hybridisierungsbedingungen sind z. B. Inkubation bei 65°C über Nacht in 7% SDS, 1% BSA, 1 mM EDTA, 250 mM Natriumphosphatpuffer (pH 7,2) und nachfolgendes Waschen bei 65°C mit 2 x SSC; 0,1% SDS.
Zu den Derivaten der erfindungsgemäßen isolierten DNA, welche gemäß Alternative F1) oder F2) durch Substitution, Addition, Inversion und/oder Deletion einer oder mehrerer Basen einer Sequenz nach einer der Gruppen A1) bis E1) und A2) bis E2) erhalten werden können, gehören insbesondere solche Sequenzen, welche in dem Protein, welches sie kodieren, zu konservativen Aminosäureaustauschen, wie z. B. dem Austausch von Glycin gegen Alanin oder von Asparaginsäure gegen Glutaminsäure führen. Solche funktionsneutralen Mutationen werden als Sinnmutationen (sense mutations) bezeichnet und führen zu keiner grundsätzlichen Veränderung der Aktivität des Polypeptids. Weiterhin ist bekannt, dass Änderungen am N- und/oder C-Terminus eines Polypeptids dessen Funktion nicht wesentlich beeinträchtigen oder dieses sogar stabilisieren können, so dass dementsprechend auch DNA-Sequenzen, bei denen am 3'-Ende oder am 5'-Ende der Sequenz mit den erfindungsgemäßen Nukleinsäuren Basen angefügt sind, von der vorliegenden Erfindung umfasst sind. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie.

Die erfindungsgemäße Nukleinsäure ist bevorzugt ein Vektor, insbesondere ein Expressionsvektor oder eine Genüberexpressionskassette. Als Vektoren kommen alle dem Fachmann bekannten Vektoren in Betracht, die üblicherweise zum Einschleusen von DNA in eine Wirtzelle eingesetzt werden. Diese Vektoren können sowohl autonom replizieren, da sie Replikationsursprünge, wie etwa jenen des 2µ-Plasmids oder ARS (autonom replizierende Sequenzen) besitzen oder in die Chromosomen integrieren (nicht-replikative Plasmide). Unter Vektoren werden auch lineare DNA-Fragmente verstanden, die keinerlei Replikationsursprünge besitzen, wie etwa Geninsertions- oder Genüberexpressionskassetten. Genüberexpressionskassetten bestehen üblicherweise aus einem Marker, den überzuexprimierenden Genen sowie für die Expression der Gene relevanten regulatorischen Bereichen, wie etwa Promotoren und Terminatoren. Optional können Genüberexpressionskassetten auch spezifische DNA-Sequenzen umfassen, welche über homologe Rekombinationsmechanismen eine zielgerichtete Integration ins Wirtsgenom ermöglichen. Je nach Aufbau der Genüberexpressionskassetten können diese bevorzugt in einfacher oder multipler Form ins Wirtsgenom integriert werden.
Bevorzugte Vektoren sind ausgewählt aus der Gruppe umfassend Plasmide und Kassetten, wie etwa *E*. *coli*-Hefe-Shuttle-Plasmide, besonders bevorzugt sind Expressionsvektoren, Geninsertions- oder Genüberexpressionskassetten.

Einen Beitrag zur Lösung der eingangs genannten Aufgabe leisten die im Folgenden beschriebenen Zellen, welche sich vorteilhaft zur Herstellung von acetylierten Sphingoidbasen, insbesondere acetyliertem Phyosphingosin, verwenden lassen. Die erfindungsgemäßen Zellen können beispielsweise in einer Biotransformation mit exogen bereitgestellter Sphingoidbase in Kontakt gebracht werden, welche die Zellen dann mit Hilfe ihrer Enzymaustattung acetylieren, oder aber es werden Zellen als Ausgangsstämme zur Herstellung der erfindungsgemäßen Zellen eingesetzt, welche selber bereits die zu acetylierenden Sphingoidbasen produzieren.
Gegenstand der vorliegenden Erfindung ist somit eine Zelle, bevorzugt eine isolierte Zelle, dadurch gekennzeichnet, dass sie derart gentechnisch verändert wurde, dass sie verglichen zu ihrem Wildtyp eine gesteigerte Aktivität mindestens eines der Enzyme E₁ und/oder E₂ aufweist, wobei das Enzym E₁ ausgewählt ist aus
einem Enzym E₁ mit der Polypeptidsequenz Seq ID Nr. 2 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 2 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 2 besitzt, wobei unter enzymatischer Aktivität für ein Enzym E₁ die Fähigkeit verstanden wird, Phytosphingosin durch Übertragung der Acetylreste aus drei Molekülen Acetyl Coenzym A zu Triacetylphytosphingosin umzusetzen,
und das Enzym E₂ ausgewählt ist aus
einem Enzym E₂ mit der Polypeptidsequenz Seq ID Nr. 4 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 4 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 4 besitzt, wobei unter enzymatischer Aktivität für ein Enzym E₂ die Fähigkeit verstanden wird, Triacetylphytosphingosin durch Übertragung des Acetylrestes aus einem Molekül Acetyl Coenzym A zu Tetraacetylphytosphingosin umzusetzen,
wobei die Steigerung der enzymatischen Aktivität durch eine erhöhte Kopiezahl der Genseguenz, welche für das Enzym kodiert, erzielt wird.

Mit "Wildtyp" einer Zelle wird hierin eine Zelle bezeichnet, deren Genom in einem Zustand vorliegt, wie er natürlicherweise durch die Evolution entstanden ist. Der Begriff wird sowohl für die gesamte Zelle als auch für einzelne Gene verwendet. Unter den Begriff "Wildtyp" fallen daher insbesondere nicht solche Zellen bzw. solche Gene, deren Gensequenzen zumindest teilweise durch den Menschen mittels rekombinanter Verfahren verändert worden sind.

Der Begriff "gesteigerte Aktivität eines Enzyms" ist vorzugsweise als gesteigerte intrazelluläre Aktivität zu verstehen.
Es ist dem Fachmann offensichtlich, dass man in Zusammenhang mit dem Begriff "verglichen zu ihrem Wildtyp veränderte (oder gesteigerte oder verminderte) Aktivität" Zellen bzw. Zellpopulationen vergleichend betrachtet, die sich in identischen, bzw. vergleichbaren Zuständen befinden, etwa bezüglich der Wachstumsphase, Kulturalter und Kultivierungsphase.

Die nun folgenden Ausführungen zur Erhöhung der Enzymaktivität in Zellen gelten sowohl für die Erhöhung der Aktivität des Enzyms E₁ bis E₂ als auch für alle nachfolgend genannten Enzyme, deren Aktivität gegebenenfalls erhöht werden kann. Grundsätzlich lässt sich eine Steigerung der enzymatischen Aktivität dadurch erzielen, dass man die Kopiezahl der Gensequenz bzw. der Gensequenzen erhöht, welche für das Enzym kodieren.

Gentechnisch veränderte Zellen werden beispielsweise durch Transformation, Transduktion, Konjugation oder eine Kombination dieser Methoden mit einem Vektor erzeugt, der das gewünschte Gen, ein Allel dieses Gens oder Teile davon und gegebenenfalls einen die Expression des Gens ermöglichenden Promotor enthält. Die heterologe Expression wird insbesondere durch Integration des Gens oder der Allele in das Chromosom der Zelle oder einen extrachromosomal replizierenden Vektor erzielt.

Die Expression der vorstehend und aller nachfolgend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar. Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei coryneformen Bakterien und zur Identifizierung der Proteine ist die von Hermann et al. (Electrophoresis, 22: 1712.23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschliessende optische Auswertung mit entsprechender Software zur Konzentrationsbestimmung (Lohaus und Meyer (1989) Biospektrum, 5: 32-39; Lottspeich (1999) Angewandte Chemie 111: 2630-2647) analysiert werden. Die Aktivität von DNA-bindenden Proteinen kann mittels DNA-Band-Shift-Assays (auch als Gelretardation bezeichnet) gemessen werden (Wilson et al. (2001) Journal of Bacteriology, 183: 2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: a laboratory manual, 2nd Ed. Cold Spring Harbor Laboratory Press, Cold Spring Harbor, N.Y. USA, 1989). Die intrazellulären enzymatischen Aktivitäten können nach verschiedenen beschriebenen Methoden (Donahue et al. (2000) Journal of Bacteriology 182 (19): 5624-5627; Ray et al. (2000) Journal of Bacteriology 182 (8): 2277-2284; Freedberg et al. (1973) Journal of Bacteriology 115 (3): 816-823) bestimmt werden. Sofern in den nachfolgenden Ausführungen keine konkreten Methoden zur Bestimmung der Aktivität eines bestimmten Enzyms angegeben werden, erfolgt die Bestimmung der Steigerung der Enzymaktivität und auch die Bestimmung der Verminderung einer Enzymaktivität vorzugsweise mittels der in Hermann et al., Electophoresis, 22: 1712-23 (2001), Lohaus et al., Biospektrum 5 32-39 (1998), Lottspeich, Angewandte Chemie 111: 2630-2647 (1999) und Wilson et al., Journal of Bacteriology 183: 2151-2155 (2001) beschriebenen Methoden.

Zur Erhöhung der Expression der jeweiligen Gene werden zum Beispiel episomale oder integrative Plasmide eingesetzt. Als Plasmide bzw. Vektoren kommen im Prinzip alle dem Fachmann für diesen Zweck zur Verfügung stehenden Ausführungsformen in Frage. Derartige Plasmide und Vektoren können z. B. den Broschüren der Firmen Novagen, Promega, New England Biolabs, Clontech oder Gibco BRL entnommen werden. Weitere bevorzugte Plasmide und Vektoren können gefunden werden in: Glover, D. M. (1985) DNA cloning: a practical approach, Vol. I-III, IRL Press Ltd. , Oxford; Rodriguez, R.L. und Denhardt, D. T (eds) (1988) Vectors : a survey of molecular cloning vectors and their uses, 179-204, Butterworth, Stoneham; Goeddel, D. V. (1990) Systems for heterologous gene expression, Methods Enzymol. 185, 3-7; Sambrook, J.; Fritsch, E. F. und Maniatis, T. (1989), Molecular cloning: a laboratory manual, 2nd ed., Cold Spring Harbor Laboratory Press, New York.

Der Plasmidvektor, der das zu amplifizierende Gen enthält, wird anschließend durch Konjugation oder Transformation in den gewünschten Stamm überführt. Die Methode der Konjugation ist beispielsweise bei Schäfer et al., Applied and Environmental Microbiology 60: 756-759 (1994) beschrieben. Methoden zur Transformation sind beispielsweise bei Schorsch et al., Current Genetics 55(4): 381-389 (2009), Thierbach et al., Applied Microbiology and Biotechnology 29: 356-362 (1988), Dunican und Shivnan, Bio/Technology 7: 1067-1070 (1989) und Tauch et al., FEMS Microbiology Let-ters 123: 343-347 (1994) beschrieben. Im Falle von integrativen Plasmiden oder linearen Genüberexpressionskassetten werden diese entweder ektopisch (nicht homolog) oder zielgerichtet durch homologe Rekombinationsmechanismen ("crossing over") ins Genom des Wirtsstammes integriert. Abhängig vom genauen Aufbau des Plasmids bzw. der Genüberexpressionskassette und von dem jeweiligen Rekombinationsereignis enthält der resultierende Stamm eine oder mehrere Kopien des betreffenden Gens.

Unter der vorstehend und in den nachfolgenden Ausführungen verwendeten Formulierung "eine im Vergleich zu ihrem Wildtyp gesteigerte Aktivität eines Enzyms Eₓ" ist vorzugsweise stets eine um einen Faktor von mindestens 2, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Aktivität des jeweiligen Enzyms Eₓ zu verstehen. Weiterhin umfasst die erfindungsgemäße Zelle, welche "eine gegenüber ihrem Wildtyp gesteigerte Aktivität eines Enzyms Eₓ" aufweist, insbesondere auch eine Zelle, deren Wildtyp keine oder zumindest keine nachweisbare Aktivität dieses Enzyms Eₓ aufweist, und die erst nach Erhöhung der Enzymaktivität, beispielsweise durch Überexpression, eine nachweisbare Aktivität dieses Enzyms Eₓ zeigt. In diesem Zusammenhang umfasst der Begriff "Überexpression" oder die in den nachfolgenden Ausführungen verwendete Formulierung "Erhöhung der Expression" auch den Fall, dass eine Ausgangszelle, beispielsweise eine Wildtyp-Zelle, keine oder zumindest keine nachweisbare Expression aufweist und erst durch rekombinante Verfahren eine nachweisbare Synthese des Enzyms Eₓ induziert wird.

Änderungen von Aminosäureresten einer gegebenen Polypeptidsequenz, die zu keinen wesentlichen Änderungen der Eigenschaften und Funktion des gegebenen Polypeptides führen, sind dem Fachmann bekannt. So können beispielsweise sogenannte konservierte Aminosäuren gegeneinander ausgetauscht werden; Beispiele für solche geeigneten Aminosäuresubstitutionen sind: Ala gegen Ser; Arg gegen Lys; Asn gegen Gln oder His; Asp gegen Glu; Cys gegen Ser; Gln gegen Asn; Glu gegen Asp; Gly gegen Pro; His gegen Asn oder Gln; Ile gegen Leu oder Val; Leu gegen Met oder Val; Lys gegen Arg oder Gln oder Glu; Met gegen Leu oder Ile; Phe gegen Met oder Leu oder Tyr; Ser gegen Thr; Thr gegen Ser; Trp gegen Tyr; Tyr gegen Trp oder Phe; Val gegen Ile oder Leu. Ebenso ist bekannt, dass Änderungen besonders am N- oder C-Terminus eines Polypeptides in Form von beispielsweise Aminosäureinsertionen oder-deletionen oft keinen wesentlichen Einfluss auf die Funktion des Polypeptides ausüben.

Die Aktivität des Enzyms E₁ wird bestimmt wie in Barenholz and Gatt, The Journal of Biological Chemistry 247 (21): 6827-6833 (1972) beschrieben, wobei als Substrat Phytosphingosin eingesetzt und dessen Acetylierung verglichen zu einem bis auf die Eigenschaft "als Enzym E₁ enthaltend ein Enzym mit Seq ID Nr. 2" identischen Referenzsystem vermessen wird.

Die Aktivität des Enzyms E₂ wird bestimmt wie in Barenholz and Gatt, The Journal of Biological Chemistry 247 (21): 6827-6833 (1972) beschrieben, wobei als Substrat triacetyliertes Phytosphingosin eingesetzt und dessen Acetylierung verglichen zu einem bis auf die Eigenschaft "als Enzym E₂ enthaltend ein Enzym mit Seq ID Nr. 4" identischen Referenzsystem vermessen wird.

Erfindungsgemäß bevorzugte Zellen weisen eine verstärkte Aktivität beider Enzyme E₁ und E₂ auf.

In einer bevorzugten alternative der Erfindung sind für die Herstellung von Triacetyl Phytosphingosin sowie den diacetylierten Sphingoidbasen Diacetyl Sphingosin, Diacetyl Sphinganin, Diacetyl -6-Hydroxysphingosin und Diacetyl Sphingadienin, Zellen nützlich, die über eine verglichen zu ihrem Wildtyp verminderte Aktivität des Enzyms E₂ verfügen. In diesem Zusammenhang sind insbesondere Zellen von Nutzen, die neben der verminderten Aktivität des Enzyms E₂ über eine verstärkte Aktivität des Enzyms E₁ verfügen.

Erfindungsgemäß bevorzugte Zellen sind Mikroorganismen, insbesondere Hefen oder Bakterien, wobei bevorzugte Hefezellen insbesondere ausgewählt sind aus den Gattungen *Saccharomyces, Pichia, Yarrowia, Kluyveromyces, Hansenula, Ashbya* und *Candida* , wobei die Arten *Saccharomyces cerevisiae, Kluyveromyces lactis, Hansenula polymorpha, Pichia pastoris, Pichia ciferrii, Yarrowia lipolytica, Candida albicans, Candida utilis* und *Ashbya gossypii* besonders bevorzugt sind.

Es ist insbesondere erfindungsgemäß bevorzugt, wenn zur Herstellung der erfindungsgemäßen Zellen bereits Ausgangsstämme eingesetzt werden, welche über einen hohen Sphingoidbasen Titer verfügen; damit leiten sich erfindungsgemäß bevorzugte Zellen insbesondere von den in der WO2006048458, WO2007131720 und der DE102011110959.9 beschriebenen Zellen sowie von Stämmen ausgewählt aus der Gruppe bestehend aus *Pichia ciferrii NRRL Y-1031 F-60-10* (Wickerham and Stodola, Journal of Bacteriology 80: 484-491 (1960), den in den Beispielen der WO 95/12683 offenbarten *Pichia* ciferrii-Stämmen und dem Stamm *Pichia ciferri* CS.PCΔPro2, beschrieben in Schorsch et al., 2009, Curr Genet. 55, 381-9, ab.

Ein weiteren Beitrag zur Lösung der der Erfindung gestellten Aufgabe leistet die Verwendung der erfindungsgemäßen Zellen zur Herstellung von acetylierten Sphingoidbasen.
Unter dem Begriff "Sphingoidbasen" im Zusammenhang mit der vorliegenden Erfindung sind Phytosphingosin, Sphingosin, Sphingadienin, 6-Hydroxysphingosin und Sphinganin (Dihydrosphingosin), auch in der acetylierten Form, wie z.B. Tetraacetylphytosphingosin, Triacetylphytosphingosin, Diacetylphytosphingosin,O-Acetylphytosphingosin, Triacetylsphinganin, Diacetylsphinganin, O-Acetylsphinganin, Triacetylsphingosin, Diacetylsphingosin, O-Acetylsphingosin, Tetraacetyl-6-Hydroxysphingosin, Triacetyl-6-Hydroxysphingosin, Diacetyl-6-Hydroxysphingosin, O-Acetyl-6-Hydroxysphingosin, Triacetylsphingadienin, Diacetylsphingadienin, O-Acetylsphingadienin zu verstehen.

Insbesondere die Verwendung der erfindungsgemäßen Zellen zur Herstellung von Sphingoidbasen und/oder Sphingolipiden ausgewählt aus der Gruppe, Tetraacetylphytosphingosin (TAPS), Triacetylphytosphingosin, Diacetylphytosphingosin, O-Acetylphytosphingosin, N-Acetylphytosphingosin, Triacetylsphinganin (TriASa), Diacetylsphinganin, O-Acetylsphinganin, N-Acetylsphinganin, Triacetylsphingosin (TriASo), Diacetylsphingosin, O-Acetylsphingosin, N-Acetylsphingosin, Tetraacetyl-6-Hydroxysphingosin, Triacetyl-6-Hydroxysphingosin, Diacetyl-6-Hydroxysphingosin, O-Acetyl-6-Hydroxysphingosin, N-Acetyl-6-Hydroxysphingosin, Triacetylsphingadienin, Diacetylsphingadienin, O-Acetylsphingadienin, N-Acetylsphingadienin ist vorteilhaft. Ganz besonders bevorzugt ist die Verwendung der erfindungsgemäßen Zellen zur Herstellung von Tetraacetylphytosphingosin (TAPS).
Eine erfindungsgemäß bevorzugte Verwendung ist erfindungsgemäß dadurch gekennzeichnet, dass erfindungsgemäß bevorzugte, wie oben beschriebene Zellen verwendet werden.

Einen weiteren Beitrag zur Lösung der der Erfindung gestellten Aufgabe leistet ein Verfahren zur Herstellung von acetvlierten Sphingoidbasen umfassend die Verfahrensschritte
a) In Kontakt Bringen der erfindungsgemäßen Zelle mit einem Medium beinhaltend eine Kohlenstoffquelle,
b) Kultivieren der Zelle unter Bedingungen, die es der Zelle ermöglichen aus der Kohlenstoffquelle Sphingoidbasen zu bilden und
c) gegebenenfalls Isolierung der gebildeten acetvlierten Sphingoidbasen.

Erfindungsgemäß bevorzugte Verfahren setzen oben als erfindungsgemäß bevorzugt genannte Zellen ein.
Als Kohlenstoffquelle können Kohlehydrate wie z. B. Glukose, Fruktose, Glycerin, Saccharose, Maltose, Melasse, aber auch Alkohole wie z.B. Ethanol organische Säuren wie z.B. Acetat eingesetzt werden. Als Stickstoffquelle können z.B. Ammoniak, Ammoniumsulfat, Ammoniumnitrat, Ammoniumchlorid, organische Stickstoffverbindungen (wie Hefeextrakt, Malzextrakt, Pepton, Maisquellwasser) eingesetzt werden. Des Weiteren können anorganische Verbindungen wie z.B. Phosphat-, Magnesium-, Kalium-, Zink-, Eisensalze und andere eingesetzt werden. Geeignete Kultivierungsbedingungen, die es der Zelle ermöglichen aus der Kohlenstoffquelle Sphingoidbasen und/oder Sphingolipide zu bilde, sind dem Fachmann für *Pichia ciferri* beispielsweise aus der WO2006048458 und der WO2007131720 bekannt. Diese Bedingungen kann das Fachmann ohne experimentellen Bedarf auf andere Zelltypen übertragen.
Das erfindungsgemäße Verfahren ist insbesondere zur Herstellung von Tetraacetylphytosphingosine (TAPS) geeignet, insbesondere wenn Zellen eingesetzt werden, die eine verstärkte Aktivität beider Enzyme E₁ und E₂ aufweisen.

Die beschriebenen Enzyme lassen sich ebenfalls vorteilhaft zur Acetylierung der Aminogruppen aliphatischer primärer Amine mit 6 bis 18 C-Atomen wie z.B. Hexadecylamin nutzen.
Somit ist ein weiterer Gegenstand der vorliegenden Erfindung ein Verfahren zur Herstellung von N-acetylierten, primären, aliphatischen Aminen umfassend die Verfahrensschritte
A) In Kontakt Bringen mindestens eines der Enzyme E₁ oder E₂, wobei das Enzym E₁ ausgewählt ist aus einem Enzym E₁ mit der Polypeptidsequenz Seq ID Nr. 2 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 2 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 2 besitzt, wobei unter enzymatischer Aktivität für ein Enzym E₁ die Fähigkeit verstanden wird, Phytosphingosin durch Übertragung der Acetylreste aus drei Molekülen Acetyl Coenzym A zu Triacetylphytosphingosin umzusetzen,
   und das Enzym E₂ ausgewählt ist aus
   einem Enzym E₂ mit der Polypeptidsequenz Seq ID Nr. 4 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 4 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 4 besitzt, wobei unter enzymatischer Aktivität für ein Enzym E₂ die Fähigkeit verstanden wird, Triacetylphytosphingosin durch Übertragung des Acetylrestes aus einem Molekül Acetyl Coenzym A zu Tetraacetylphytosphingosin umzusetzen.
   mit einem Medium beinhaltend ein primäres, aliphatisches Amin, insbesondere ausgewählt aus solchen, welche 6 bis 18 Kohlenstoffatome aufweisen, welche bevorzugt linear sind,
   und Acetyl-CoA,
B) gegebenenfalls Isolierung der gebildeten acetylierten Amine, wobei als Enzyme E₁ und/oder E₂ oben als erfindungsgemäß genannte Zellen eingesetzt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Überexpression von PcSLI1 in der Hefe Saccharomyces cerevisiae Stamm K26

Für die Überexpression von *PcSLI1* in *Saccharomyces cerevisiae* Stamm K26 wurde der *PcSLI1-*ORF zunächst in den 2µ-Vektor p426HXT7-6HIS (Hamacher et al. Microbiology 148: 2783-2788 (2002), (Seq ID Nr. 6)) kloniert. Der *PcSLI1-*ORF wurde hierfür durch Polymerase-Kettenreaktion (PCR) amplifiziert. Zunächst wurde genomische DNA durch eine abgewandelte Cethyltrimethylammoniumbromid-(CTAB) Methode (Murray and Thompson; Nucleic Acids Res 8, 4321-4325 (1980)) aus P. *ciferrii* isoliert. Hierzu wurden Zellen einer in YEPD-Flüssigmedium (1% w/v Hefeextrakt, 1% w/v Pepton, 2% w/v Glukose) angezogenen Kultur (≥ 2 ml, OD₆₀₀ₙₘ > 1) geerntet, in 400 µl CTAB-Puffer [2 % (w/v) CTAB, 100 mM Tris-HCl (pH 8.0), 20 mM EDTA, 1,4 M NaCl] resuspendiert, mit 200 µl Glasperlen (0,25 - 0,5 mm ∅) versetzt und 8 min bei 4° C auf einem "Vibrax VXR basic" (2200 rpm) aufgeschlossen. Anschließend wurde der Ansatz für 30 min bei 65° C inkubiert. Nach Zugabe einen Volumens Chloroform und anschließendem Homogenisieren für 10 Sek. wurde der Ansatz für 5 min bei 16.000 g zentrifugiert. Der DNA-haltige Überstand wurde entnommen und die DNA für mindestens 30 min bei -20° C mit 0,7 Volumen Isopropanol gefällt. Nach der folgenden Zentrifugation für 15 min bei 16.000 g und 4° C wurde das Sediment mit 70 %igem eiskalten Ethanol gewaschen, getrocknet und in 50 µl H₂O resuspendiert.

Die PCR-Amplifizierung des *PcSLI1*-ORFs erfolgte dann mit genomischer *P.ciferrii-*DNA als Template und mit den beiden Oligonukleotiden SLI1.HXT7.fw (Seq ID Nr. 11) und SLI1.CYC1.rv (Seq ID Nr. 12). Die verwendeten Primer besaßen dabei jeweils am 5'-Ende Bereiche, die homolog zu dem Integrationsbereich in den Zielvektor waren. In S. *cerevisiae* ermöglichen diese homologen Enden eine homologe Rekombination zwischen einem linearisierten Vektor und PCR-Fragmenten, um ein zirkuliertes Plasmid zu erzeugen, welches *in vivo* vermehrt werden kann. Als DNA-Polymerase wurde die Phusion™ High-Fidelty DNA -Polymerase (Finnzymes) gemäß Herstellerangaben eingesetzt. Zur Amplifikation wurde folgendes Temperaturprofil gewählt: Schritt 1: 98° C, 2 min (Denaturierung); Schritt 2: 98° C, 15 sek (Denaturierung); Schritt 3: 60° C, 25 sek (Annealing); Schritt 4: 72° C, 80 sek (Elongation); Schritt 5: 72° C, 5 min (Elongation). Die Schritte 2-4 wurden 35 x wiederholt. Das resultierende 1,4 kb PCR-Fragment wurde nach Agarose-Gelelektrophorese mittels "NucleoSpin® Extract II"-Gelextraktionskit (Macherey-Nagel) nach Herstellerangaben aufgereinigt.

Das Plasmid p426HXT7-6HIS (Seq ID Nr. 6) wurde nach Herstellerangaben mit BamHI/EcoRI verdaut. Das resultierende 6,3 kb Vektorfragment wurde ebenfalls nach Agarose-Gelelektrophorese mittels "NucleoSpin® Extract II"-Gelextraktionskit (Macherey-Nagel) nach Herstellerangaben aufgereinigt.

Die Klonierung des mittels PCR amplifizierten *PcSLI1*-ORFs in den mittels BamHI/EcoRI geschnittenen Vektor erfolgte durch in vivo Rekombination in S. *cerevisiae.* Das grundsätzliche Verfahren ist in Oldenburg et al. (Nucleic Acids Res 25: 451-452 (1994)) beschrieben. Die beiden gereinigten DNA-Fragmente wurden zusammen in S. *cerevisiae* CEN.PK113-13D (K26) transformiert, wobei das Protokoll von Gietz und Schiestl (Nat Protoc 2: 31-34 (2007)) befolgt wurde. Die Zellen wurden im Anschluss auf synthetischem Minimalmedium (0,16% w/v Yeast Nitrogen Base, 0,5% w/v Ammoniumsulfat, 2% w/v Glucose, 2% w/v Agar) ausplattiert. Dadurch konnte auf Transformanten selektiert werden, die aufgrund homologer Rekombination des DNA-Fragmentes mit dem linearisierten Vektor ein stabiles, zirkularisiertes Plasmid besaßen. Plasmide wurden nachfolgend aus den Hefeklonen isoliert. Dazu wurden Zellen einer 2 ml Kultur angezogen in synthetischem Minimalmedium (OD₆₀₀ₙₘ > 1) geerntet, gewaschen und in 400 µl Puffer 1 [50 mM Glukose; 10 mM EDTA (Titriplex III); 25 mM Tris-HCl (pH 8); RNase A (100 µg/ml)] resuspendiert. Nach Zugabe von 400 µl Puffer 2 (0,2 M NaOH; 1 % SDS) und vorsichtigem Mischen, wurde ca. ²/₃ des Volumens an Glasperlen (0,25 - 0,5 mm O) hinzugegeben und die Zellen bei 4° C für 8 min auf einem "Vibrax VXR basic" bei 2200 rpm aufgeschlossen. 500 µl Überstand wurden mit 250 µl Puffer 3 [5 M Kalium-Acetat (pH 5,5)] vermischt, 10 min auf Eis inkubiert und 5 min bei 16.000 g zentrifugiert. Der Überstand wurde für mindestens 30 min bei -20° C mit Isopropanol im Verhältnis 1:1 gefällt und anschließend 20 min bei 16.000 g zentrifugiert. Die pelletierte DNA wurde mit 70 % Ethanol (-20° C) gewaschen und in 50 µl Wasser gelöst. Nachfolgend wurde die Plasmid-DNA per Elektroporation nach Dower et al. (Nucleic Acids Res 16: 6127-6145 (1988)) in *E. coli* transformiert. Für die Elektroporation wurde der Gene Pulser® unter folgenden Bedingungen verwendet: Spannung: 2 - 2,5 kV; Widerstand: 200 Ω; Kapazität: 25 µF. Transformanten wurden auf festem LB-Medium (1% w/v Trypton, 0,5% w/v Hefeextrakt, 0,5% w/v NaCl, 2% Agar, pH 7,5) supplementiert mit 40 µg/ml Ampicillin selektioniert. Für die Isolation der Plasmide aus *E. coli* wurden die Klone in 5 ml flüssigem LB-Medium supplementiert mit 40 µg/ml Ampicillin über Nacht bei 37° C auf einem Schüttler angezogen, nachfolgend wurde das GeneJET™ Plasmid Miniprep Kit (Fermentas GmbH) nach Herstellerangaben eingesetzt.

Die Plasmide wurden durch eine anschließende Restriktionsanalyse und Sequenzierung charakterisiert. Die korrekte Integration des *PcSLI1*-ORFs in den linearisierten Vektor ergab das 7,6 kb Plasmid pCS.426.SL11 (Seq ID Nr. 7), bei dem der *PcSLI1-*ORF unter Kontrolle des verkürzten *HXT7³⁹²⁻¹* Promotor-Fragments und des *CYC1*-Terminators aus S. *cerevisiae* steht. Diese Anordnung ermöglicht eine konstitutive Überexpression von *PcSLI1* in *S. cerevisiae.*

Die Plasmide pCS.426.SL11 (Seq ID Nr. 7) sowie das Kontrollplasmid p426HXT7-6HIS (Seq ID Nr. 6) wurden nachfolgend in S. *cerevisiae* Stamm K26 transformiert, wobei wieder die Methode von Gietz und Schiestl (Nat Protoc 2: 31-34 (2007)) befolgt wurde. Transformanten wurden abermals auf synthetischem Minimalmedium (0,16% w/v Yeast Nitrogen Base, 0,5% w/v Ammoniumsulfat, 2% w/v Glucose, 2% w/v Agar) selektioniert. Nachfolgend wurden die Transformanten in flüssigem TAPS-Medium [Zusammensetzung pro Liter: 33 g Glukose-Monohydrat, 20 g Kalium-Hydrogenphthalat, 4.83 g Ammonium-Chlorid, 1 g Hefeextrakt, 1 g KaliumDihydrogenphosphat, 0.88 g Magnesiumsulfat-Heptahydrat, 0.2 g Kalciumchlorid-Dihydrat, 60 mg Natrium-Chlorid, 59 mg myo-Inositol, Spurenelemente [37.3 mg Ammonium-Eisen(II)Sulfat-Hexahydrat, 7.5 mg Kupfer(II)Sulfat-Pentahydrat, 5 mg Zinksulfat-Heptahydrat, 1.5 mg Kaliumiodid, 0.6 mg Mangan(II)Sulfat-Monohydrat, 0.6 mg Borsäure, 0.6 mg Natriummolybdat-Dihydrat], Vitamine (3 mg Nikotinsäure, 3 mg Kalcium D-Pantothenat, 3 mg Thiamin, 2 mg 4-Aminobenzoesäure, 0.3 mg Pyridoxin, 10 µg Biotin); pH 5,4] kultiviert, um den Effekt der Überexpression von *PcSLI1* auf die Produktion acetylierter Sphingoidbasen zu untersuchen. Die Anzucht der Transformanten erfolgte aerob bei 30° C auf einem Rotations-Schüttelgerat bei 200 - 250 rpm. Die Zellen wurden zunachst in 5 ml TAPS-Medium als Vorkultur gezüchtet und beim Erreichen der stationären

Wachstumsphase zur Inokulation von 20 ml TAPS-Medium (≙ Hauptkultur) verwendet. Hauptkulturen wurden mit einer OD₆₀₀nm von 0,1 gestartet. Zur Analyse von acetylierten Sphingoidbasen mittels LC-MS/MS wurden bei Erreichen der Stationärphase 1 ml Proben der Kultivierungsbrühe entnommen und bis zur weiteren Aufarbeitung bei -20° C gelagert. Die Extraktion der Lipide erfolgte nach der Methode von Bjerve et al. (Anal. Biochem. 58: 238-245 (1974)). Hierfür wurden 20 µl Kulturbrühe in dem zehnfachen Volumen n-Butanol aufgenommen. 20 ng ungradzahliges C17-Phytosphingosin, C17-Sphinganin und Deuterium-markiertes d₉ Triacetylsphinganin wurden als interne Standards zugegeben, und die Probe wurde kräftig bei 70° C gemischt. Nachfolgend wurde die Probe bei 1000 x g für 5 min zentrifugiert, 10 µl des Überstandes wurden abgenommen und zehnfach in 50/50% (v/v) Methanol/H₂O verdünnt.

Für die LC-MS/MS Analyse der Sphingolipide wurde eine Thermo Accela HPLC (Thermo Fisher Scientific Inc., Waltham, MA, USA) Anlage eingesetzt. Das injizierte Probevolumen betrug 5 µl. Bei der eingesetzen Säule handelte es sich um eine Reversed-Phase Hypersil Gold C18 Säule, temperiert auf 40° C (1.9 µm Partikel; 50 x 2.1 mm; #25002-052130; Thermo Fisher Scientific Inc., Waltham, MA, USA) und geschützt durch eine Microbore Guard Säule (Nucleodur C18 ISIS; 3 µm Partikel; 5 x 1 mm; #717759; Macherey-Nagel, Dueren, Germany). Die mobile Phase hatte eine Flußrate von 200 µl/min und bestand aus (A) H₂O mit 0,1% v/v Ameisensäure und 2% v/v Acetonitril, und (B) Acetonitril mit 0,1% v/v Ameisensäure. Nach eingangs 1 min mit 70% B wurde ein 4-Schritt Gradient zur Elution genutzt: 1) Erhöhung von 70% B auf 94% über 1,5 min; 2) Erhöhung von 94% B auf 100% über 3,5 min; 3) Beibehalten von 100% B über 3,5 min; 4) Absenken von 100% B auf 70% und Beibehalten für 2,5 min. Die MS/MS Analyse wurde als *Multiple Reaction Monitoring* (MRM) im positiven lonisierungsmodus durchgeführt. Angewandte Massenübergänge und Kollisionsenergien sind in Tabelle 1 gezeigt.

**Tabelle 1. Massenübergänge und Kollisionsenergien. IS, Interner Standard (deuteriert bzw. Sphingolipid mit ungerader Kettenlänge). Fettgedruckte Werte wurden als Qualifier genutzt.**

| | Vorstufen-Ion (*m*/*z*) | Produkt-Ion(en) (*m*/*z*) | Kollisionsenergie (eV) |
|---|---|---|---|
| Sphinganin | 302.3 | 284.4/**266.4** | 10/**25** |
| C17-Sphinganin IS | 288.3 | 270.4/**252.4** | 10/**25** |
| Phytosphingosin | 318.3 | 300.4/**286.4** | 11/**25** |
| C17-Phytosphingosin IS | 304.3 | 286.4/**250.4** | 11/**25** |
| Triacetylsphinganin | 428.3 | 368.4/**266.4** | 10/**25** |
| Triacetylphytosphingosin | 444.3 | 384.4/**264.4** | 10/**25** |
| Tetraacetylphytosphingosin | 486.3 | 426.4/**264.4** | 10/**25** |
| d₉-Triacetylsphinganin IS | 437.3 | 374.4/**266.4** | 10/**25** |

Instrumentelle Einstellungen waren wie folgt: *Kapillarspannung,* 3.5 kV; *Kapillartemperatur,* 350° C, *Declustering Voltage,* 10 V; *Sheath Gas pressure,* 5 au *(arbitrary units); Ion Sweep Gas Pressure,* 0 au; *Auxiliary Gas Pressure,* 5 au, *S-lens RF Amplitude,* 50 V; Kollisionsenergie, 10-35 eV; Argon Kollisionsgasdruck, 1.0 mTorr; Zyklusdauer, 600 ms; Auflösung von Quadrupol 1 und 3 war 0.70 (fwhm). Die Xcalibur Software Version 2.1 (Thermo Fisher Scientific Inc., Waltham, MA, USA) wurde zur Datenaufnahme und -analyse genutz.

Der Einfluss der *PcSLI1*-Expression auf die Produktion acetylierter Sphingoidbasen in *S. cerevisiae* wird in Tabelle 2 gezeigt. Deutlich erhöhte Titer waren vor allem im Fall von Diacetylsphinganin, Triacetylsphinganin und Triacetylphytosphingosin zu verzeichnen, während die gebildete Menge Tetraacetylphytosphingosin i. Vgl. zum Kontrollstamm unverändert war. Dies ist ein klares Indiz dafür, dass das PcSLl1-Protein lediglich drei Acetylreste auf Phytosphingosin oder Sphinganin übertragen kann. Die finale Acetylierung von Triacetylphytosphingosin hin zu Tetraacetylphytosphingosin kann dieses Protein jedoch nicht katalysieren.

**Tabelle 2. Produktion acetylierter und freier Sphingoidbasen in rekombinanten S. cerevisiae-Stämmen. K26L, Stamm S. cerevisiae K26 transformiert mit dem Kontrollplasmid p426HXT7-6HIS; K26SLI1, Stamm S. cerevisiae K26 transformiert mit dem PcSLI1-Überexpressionsplasmid pCS.426.SLI1; Sa, Sphinganin; DiASa, Diacetylsphinganin; TriASa, Triacetylsphinganin; Ps, Phytosphingosin; TriAPS, Triacetylphytosphingosin; TAPS, Tetraacetylphytosphingosin; die Werte zeigen die in der Fermentationsbrühe ermittelten Titer in mg/l.**

| | Sa | DiASa | TriASa | Ps | TriAPS | TAPS |
|---|---|---|---|---|---|---|
| K26L | 0.65 | 0 | 0.1 | 1.0 | 0 | 0.1 |
| K26SLI1 | 0.4 | 1.5 | 0.7 | 0.6 | 1.4 | 0.1 |

### Überexpression von PcSLI1 und PcATF1 in dem Stamm Saccharomyces cerevisiae Stamm K26

Zur vollständigen Acetylierung von Phytosphingosin zum Tetraacetylphytosphingosin in Saccharomyces cerevisiae Stamm K26 bedarf es der gleichzeitigen Überexpression von *PcSLI1* und *PcATF1.* Für die Überexpression von *PcSLI1* wird das Plasmid pCS.426.SL11 verwendet, dessen Konstruktion im vorigen Beispiel beschrieben wurde. Der hierfür eingesetzte Expressionsvektor p426HXT7-6HIS (Seq ID Nr. 6) trägt das S. *cerevisiae URA3*-Markergen. Für die Überexpression von *PcATF1* muss der *PcATF1-*ORF zunächst in einen geeigneten Expressionsverktor kloniert werden, der ein alternatives Markergen trägt. Hierfür wird das Plasmidp425HXT7-6HIS (Becker and Boles; Appl Environ Microbiol 69: 4144-4150 (2003), (Seq ID Nr. 5)) eingesetzt, welches das *S. cerevisiae LEU2*-Gen als Selektionsmarker trägt.

Zunächst wird der *PcATF1-*ORF inklusive 19 bp des *PcATF1*-Terminators mittels PCR aus genomischer *P. ciferrii-DNA* amplifiziert. Als Primer werden die Oligonukleotide ATF2-HXT.fw (Seq ID Nr. 9) und ATF2-CYC.rv (Seq ID Nr. 10) eingesetzt. Die verwendeten Primer besitzen dabei jeweils am 5'-Ende Bereiche, die homolog zu dem Integrationsbereich in den Zielvektor sind. In *S. cerevisiae* ermöglichen diese homologen Enden eine homologe Rekombination zwischen einem linearisierten Vektor und PCR-Fragmenten, um ein zirkuliertes Plasmid zu erzeugen, welches *in vivo* vermehrt werden kann.

Als DNA-Polymerase wird die Phusion™ High-Fidelty DNA -Polymerase (Finnzymes) gemäß Herstellerangaben eingesetzt. Zur Amplifikation wird folgendes Temperaturprofil gewählt: Schritt 1: 98° C, 2 min (Denaturierung); Schritt 2: 98° C, 15 sek (Denaturierung); Schritt 3: 61° C, 25 sek (Annealing); Schritt 4: 72° C, 80 sek (Elongation); Schritt 5: 72° C, 5 min (Elongation). Die Schritte 2-4 werden 35 x wiederholt. Das resultierende 1,6 kb PCR-Fragment wird nach SDS-Gelelektrophorese mittels "NucleoSpin® Extract II"-Gelextraktionskit (Macherey-Nagel) nach Herstellerangaben aufgereinigt.

Das Plasmid p425HXT7-6HIS (Seq ID Nr. 5) wird nach Herstellerangaben mit BamHI/HindIII verdaut. Das resultierende 7,5 kb Vektorfragment wird ebenfalls nach SDS-Gelelektrophorese mittels "NucleoSpin® Extract II"-Gelextraktionskit (Macherey-Nagel) nach Herstellerangaben aufgereinigt.

Die Klonierung des mittels PCR amplifizierten *PcATF1-*ORFs in den mittels BamHI/EcoRI geschnittenen Vektor erfolgt durch in vivo Rekombination in *S. cerevisiae.* Das grundsätzliche Verfahren ist in Oldenburg et al. (Nucleic Acids Res 25: 451-452 (1994)) beschrieben. Die beiden gereinigten DNA-Fragmente werden zusammen in *S. cerevisiae* Stamm 10480-2C (Mösch und Fink, Genetics 145: 671-684 (1997)) transformiert, wobei das Protokoll von Gietz und Schiestl (Nat Protoc 2: 31-34 (2007)) befolgt wird. Die Zellen werden im Anschluss auf Minimalmedium (0,16% w/v Yeast Nitrogen Base, 0,5% w/v Ammoniumsulfat, 2% w/v Glucose, 2% w/v Agar) supplementiert mit 20 mg/l Uracil ausplattiert. Dadurch können Transformanten selektiert werden, die aufgrund homologer Rekombination des DNA-Fragmentes mit dem linearisierten Vektor ein stabiles, zirkularisiertes Plasmid besitzen. Plasmide werden nachfolgend aus den Hefeklonen isoliert. Dazu werden Zellen einer 2 ml Kultur angezogen in synthetischem Minimalmedium supplementiert mit 20 mg/l L-Histidin-HCl, 20 mg/l Uracil und 20 mg/l L-Tryptophan (OD₆₀₀ₙₘ > 1) geerntet, gewaschen und in 400 µl Puffer 1 [50 mM Glukose; 10 mM EDTA (Titriplex III); 25 mM Tris-HCl (pH 8); RNase A (100 µg/ml)] resuspendiert. Nach Zugabe von 400 µl Puffer 2 (0,2 M NaOH; 1 % SDS) und vorsichtigem Mischen, werden ca. ²/₃ des Volumens an Glasperlen (0,25 - 0,5 mm O) hinzugegeben und die Zellen bei 4° C für 8 min auf einem "Vibrax VXR basic" bei 2200 rpm aufgeschlossen. 500 µl Überstand werden mit 250 µl Puffer 3 [5 M Kalium-Acetat (pH 5,5)] vermischt, 10 min auf Eis inkubiert und 5 min bei 16.000 g zentrifugiert. Der Überstand wird für mindestens 30 min bei -20° C mit Isopropanol im Verhältnis 1:1 gefällt und anschließend 20 min bei 16.000 g zentrifugiert. Die pelletierte DNA wird mit 70 % Ethanol (-20° C) gewaschen und in 50 µl Wasser gelöst. Nachfolgend wird die Plasmid-DNA per Elektroporation nach Dower et al. (Nucleic Acids Res 16: 6127-6145 (1988)) in *E. coli* transformiert. Für die Elektroporation wird der Gene Pulser® unter folgenden Bedingungen verwendet: Spannung: 2 - 2,5 kV; Widerstand: 200 Ω; Kapazität: 25 µF. Transformanten werden auf festem LB-Medium (1% w/v Trypton, 0,5% w/v Hefeextrakt, 0,5% w/v NaCl, 2% Agar, pH 7,5) supplementiert mit 40 µg/ml Ampicillin selektioniert. Für die Isolation der Plasmide aus *E. coli* werden die Klone in 5 ml flüssigem LB-Medium supplementiert mit 40 µg/ml Ampicillin über Nacht bei 37° C auf einem Schüttler angezogen, nachfolgend wird das GeneJET™ Plasmid Miniprep Kit (Fermentas GmbH) nach Herstellerangaben eingesetzt.

Die Plasmide werden nachfolgend durch Restriktionsanalyse und Sequenzierung charakterisiert. Die korrekte Integration des *PcSLI1*-ORFs in den linearisierten Vektor ergibt das 8,9 kb Plasmid pCS.425.ATF1 (Seq ID Nr. 8), bei dem der *PcATF1-*ORF unter Kontrolle des verkürzten *HXT7³⁹²⁻¹* Promotor-Fragments und des *CYC1-*Terminators aus *S. cerevisiae* steht. Diese Anordnung ermöglicht eine konstitutive Überexpression von *PcATF1* in *S. cerevisiae.*

Für die zeitgleiche Überexpression von PcSLI1 und PcATF2 werden die beiden Plasmide pCS.426.SL11 (Seq ID Nr. 7) und pCS.425.ATF1 (Seq ID Nr. 8) anschließend in den *S. cerevisiae-*Stamm 10480-2C co-transformiert, wobei wiederum die Methode von Gietz und Schiestl (Nat Protoc 2: 31-34 (2007)) befolgt wird. Transformanten werden auf synthetischem Minimalmedium (0,16% w/v Yeast Nitrogen Base, 0,5% w/v Ammoniumsulfat, 2% w/v Glucose, 2% w/v Agar) selektioniert. Zu Vergleichszwecken werden auch die beiden Ausgangsplasmide p425HXT7-6HIS (Seq ID Nr. 5) und p426HXT7-6HIS (Seq ID Nr. 6) in den Stamm RH2754 cotransformiert. Nachfolgend werden die Transformanten in flüssigem TAPS-Medium kultiviert, um den Effekt der zeitgleichen Überexpression von *PcSLI1* und *PcATF1* auf die Produktion acetylierter Sphingoidbasen zu untersuchen. Die Anzucht der Transformanten erfolgt aerob bei 30° C auf einem Rotations-Schüttelgerat bei 200 - 250 rpm. Die Zellen werden zunächst in 5 ml TAPS-Medium als Vorkultur gezüchtet und beim Erreichen der stationären Wachstumsphase zur Inokulation von 20 ml TAPS-Medium (≙ Hauptkultur) verwendet. Kultufführung, Aufarbeitung der Proben und Vorgehen zur Bestimmung der Produktion acetylierter Sphingoidbasen erfolgen analog zum vorigen Beispiel.

Die Analyse zeigt im Falle des mit den beiden Plasmiden pCS.426.SL11 und pCS.425.ATF1 transformierten Hefestammes einen deutlich gesteigerten Titer an Tetraacetylphytosphingosin im Vergleich zu dem mit den beiden Kontrollplasmiden (p425HXT7-6HIS und p426HXT7-6HIS) transformierten Stamm. Dies ist ein eindeutiger Beweis dafür, dass die gleichzeitige Überexpression von *PcSLI1* und *PcATF1* in *Saccharomyces cerevisiae* eine vollständige Acetylierung des Metaboliten Phytosphingosin bewirkt.

## Patentansprüche

1. Isolierte Nukleinsäure, welche eine Sequenz ausgewählt aus den Gruppen A1) bis D1 und G1) aufweist
A1) eine Sequenz gemäß Seq ID Nr. 1, wobei diese Sequenz für ein Protein kodiert, welches in der Lage ist,
Phytosphingosin durch Übertragung der Acetylreste aus drei Molekülen Acetyl Coenzym A zu Triacetylphytosphingosin umzusetzen,
B1) eine intronfreie Sequenz, die von einer Sequenz nach A1) abgeleitet ist und die das gleiche Protein oder Peptid kodiert wie die Sequenz nach Seq ID Nr. 1,
C1) eine Sequenz, die ein Protein oder Peptid kodiert, das die Aminosäuresequenz gemäß Seq ID Nr. 2 umfasst und das in der Lage ist, Phytosphingosin durch Übertragung der Acetylreste aus drei Molekülen Acetyl Coenzym A zu Triacetylphytosphingosin umzusetzen,
D1) eine Sequenz, die mit einer Sequenz nach einer der Gruppen A1) bis C1) zu mindestens 70% identisch ist, wobei diese Sequenz für ein Protein oder Peptid kodiert, welches in der Lage ist,
Phytosphingosin durch Übertragung der Acetylreste aus drei Molekülen Acetyl Coenzym A zu Triacetylphytosphingosin umzusetzen,
G1) eine komplementäre Sequenz zu einer Sequenz nach einer der Gruppen A1) bis D1).

2. Isolierte Nukleinsäure, welche eine Sequenz ausgewählt aus den Gruppen A2) bis D2 und G2) aufweist
A2) eine Sequenz gemäß Seq ID Nr. 3, wobei diese Sequenz für ein Protein kodiert, welches in der Lage ist,
Triacetylphytosphingosin durch Übertragung des Acetylrestes aus einem Molekül Acetyl Coenzym A zu Tetraacetylphytosphingosin umzusetzen,
B2) eine intronfreie Sequenz, die von einer Sequenz nach A2) abgeleitet ist und die das gleiche Protein oder Peptid kodiert wie die Sequenz nach Seq ID Nr. 3,
C2) eine Sequenz, die ein Protein oder Peptid kodiert, das die Aminosäuresequenz gemäß Seq ID Nr. 4 umfasst, und welches in der Lage ist,
Triacetylphytosphingosin durch Übertragung des Acetylrestes aus einem Molekül Acetyl Coenzym A zu Tetraacetylphytosphingosin umzusetzen,
D2) eine Sequenz, die mit einer Sequenz nach einer der Gruppen A2) bis C2) zu mindestens 70% identisch ist, wobei diese Sequenz für ein Protein oder Peptid kodiert, welches in der Lage ist,
Triacetylphytosphingosin durch Übertragung des Acetylrestes aus einem Molekül Acetyl Coenzym A zu Tetraacetylphytosphingosin umzusetzen,
G2) eine komplementäre Sequenz zu einer Sequenz nach einer der Gruppen A2) bis D2).

3. Zelle, **dadurch gekennzeichnet, dass** sie derart gentechnisch verändert wurde,
dass sie verglichen zu ihrem Wildtyp eine gesteigerte Aktivität mindestens eines der Enzyme E₁ und/oder E₂ aufweist, wobei das Enzym E₁ ausgewählt ist aus einem Enzym E₁ mit Polypeptidsequenz Seq ID Nr. 2 oder mit einer Polypeptidsequenz, bei der bis zu 25% der Aminosäurereste gegenüber der Bezugssequenz Seq ID Nr. 2 durch Deletion, Insertion, Substitution oder eine Kombination davon verändert sind und die noch mindestens 10% der enzymatischen Aktivität des Enzyms mit der Bezugssequenz Seq ID Nr. 2 besitzt, wobei unter enzymatischer Aktivität für ein Enzym E₁ die Fähigkeit verstanden wird, Phytosphingosin durch Übertragung der Acetylreste aus drei Molekülen Acetyl Coenzym A zu Triacetylphytosphingosin umzusetzen,
und das Enzym E₂ ausgewählt ist aus
einem Enzym E₂ mit Polypeptidsequenz Seq ID Nr. 4 oder mit einer Polypeptidsequenz bei der bis zu 25% der Aminosäurereste gegenüber der Bezugssequenz Seq ID Nr. 4 durch Deletion, Insertion, Substitution oder eine Kombination davon verändert sind und die noch mindestens 10% der enzymatischen Aktivität des Enzyms mit der Bezugssequenz Seq ID Nr. 4 besitzt, wobei unter enzymatischer Aktivität für ein Enzym E₂ die Fähigkeit verstanden wird, Triacetylphytosphingosin durch Übertragung des Acetylrestes aus einem Molekül Acetyl Coenzym A zu Tetraacetylphytosphingosin umzusetzen,
wobei die Steigerung der enzymatischen Aktivität durch eine erhöhte Kopiezahl der Gensequenz, welche für das Enzym kodiert, erzielt wird.

4. Zelle gemäß Anspruch 3, **dadurch gekennzeichnet, dass** sie ausgewählt ist aus *Saccharomyces cerevisiae, Kluyveromyces lactis, Hansenula polymorpha, Pichia pastoris, Pichia ciferrii, Yarrowia lipolytica, Candida albicans, Candida utilis* und *Ashbya gossypii.*

5. Verwendung der Zellen gemäß Anspruch 3 oder 4 zur Herstellung von acetylierten Sphingoidbasen.

6. Verfahren zur Herstellung von acetylierten Sphingoidbasen umfassend die Verfahrensschritte
a) In Kontakt bringen der Zelle gemäß Anspruch 3 oder 4 mit einem Medium beinhaltend eine Kohlenstoffquelle,
b) Kultivieren der Zelle unter Bedingungen, die es der Zelle ermöglichen aus der Kohlenstoffquelle Sphingoidbasen zu bilden und
c) gegebenenfalls Isolierung der gebildeten acetylierten Sphingoidbasen.

7. Verfahren zur Herstellung von N-acetylierten, primären, aliphatischen Aminen umfassend den Verfahrensschritt
A) In Kontakt Bringen mindestens eines der Enzyme E₁ oder E₂, wobei das Enzym E₁ ausgewählt ist aus
einem Enzym E₁ mit der Polypeptidsequenz Seq ID Nr. 2 oder mit einer Polypeptidsequenz bei der bis zu 25% der Aminosäurereste gegenüber der Bezugssequenz Seq ID Nr. 2 durch Deletion, Insertion, Substitution oder eine Kombination davon verändert sind und die noch mindestens 10% der enzymatischen Aktivität des Enzyms mit der Bezugssequenz Seq ID Nr. 2 besitzt, wobei unter enzymatischer Aktivität für ein Enzym E₁ die Fähigkeit verstanden wird, Phytosphingosin durch Übertragung der Acetylreste aus drei Molekülen Acetyl Coenzym A zu Triacetylphytosphingosin umzusetzen,
und das Enzym E₂ ausgewählt ist aus
einem Enzym E₂ mit der Polypeptidsequenz Seq ID Nr. 4 oder mit einer Polypeptidsequenz bei der bis zu 25% der Aminosäurereste gegenüber der Bezugssequenz Seq ID Nr. 4 durch Deletion, Insertion, Substitution oder eine Kombination davon verändert sind und die noch mindestens 10% der enzymatischen Aktivität des Enzyms mit der Bezugssequenz Seq ID Nr. 4 besitzt, wobei unter enzymatischer Aktivität für ein Enzym E₂ die Fähigkeit verstanden wird, Triacetylphytosphingosin durch Übertragung des Acetylrestes aus einem Molekül Acetyl Coenzym A zu Tetraacetylphytosphingosin umzusetzen.
mit einem Medium beinhaltend ein primäres, aliphatisches Amin ,
und Acetyl-CoA,
**dadurch gekennzeichnet, dass** als das mindestens eine der Enzyme E₁ oder E₂ Zellen gemäß Anspruch 3 eingesetzt werden.

8. Verfahren gemäß Anspruch 7, **dadurch gekennzeichnet, dass** das primäre, aliphatische Amin ausgewählt aus solchen, welche 6 bis 18 Kohlenstoffatome aufweisen.

9. Verfahren gemäß Anspruch 7 oder 8, **dadurch gekennzeichnet, dass** es den Verfahrensschritt
B) Isolierung der gebildeten acetylierten Amine,
umfasst.

## Claims

1. Isolated nucleic acid which has a sequence selected from the groups A1) to D1 and G1)
A1) a sequence according to Seq ID No. 1, wherein this sequence codes for a protein which is capable of converting phytosphingosine to triacetylphytosphingosine by transfer of the acetyl residues from three molecules of acetyl coenzyme A,
B1) an intron-free sequence which is derived from a sequence according to A1) and which encodes the same protein or peptide as the sequence according to Seq ID No. 1,
C1) a sequence which encodes a protein or peptide which includes the amino acid sequence according to Seq ID No. 2 and which is capable of converting phytosphingosine to triacetylphytosphingosine by transfer of the acetyl residues from three molecules of acetyl coenzyme A,
D1) a sequence which is at least 70% identical with a sequence according to one of the groups A1) to C1), wherein this sequence codes for a protein or peptide which is capable of converting phytosphingosine to triacetylphytosphingosine by transfer of the acetyl residues from three molecules of acetyl coenzyme A,
G1) a complementary sequence to a sequence according to one of the groups A1) to D1).

2. Isolated nucleic acid which has a sequence selected from the groups A2) to D2 and G2)
A2) a sequence according to Seq ID No. 3, wherein this sequence codes for a protein which is capable of converting triacetylphytosphingosine to tetraacetylphytosphingosine by transfer of the acetyl residue from one molecule of acetyl coenzyme A,
B2) an intron-free sequence which is derived from a sequence according to A2) and which encodes the same protein or peptide as the sequence according to Seq ID No. 3,
C2) a sequence which encodes a protein or peptide which includes the amino acid sequence according to Seq ID No. 4 and which is capable of converting triacetylphytosphingosine to tetraacetylphytosphingosine by transfer of the acetyl residue from one molecule of acetyl coenzyme A,
D2) a sequence which is at least 70% identical with a sequence according to one of the groups A2) to C2), wherein this sequence codes for a protein or peptide which is capable of converting triacetyl-phytosphingosine to tetraacetylphytosphingosine by transfer of the acetyl residue from one molecule of acetyl coenzyme A,
G2) a complementary sequence to a sequence according to one of the groups A2) to D2).

3. Cell, **characterized in that** it has been genetically modified such that compared to the wild type thereof it exhibits increased activity of at least one of the enzymes E₁ and/or E₂, wherein the enzyme E₁ is selected from an enzyme E₁ with polypeptide sequence Seq ID No. 2 or with a polypeptide sequence in which up to 25% of the amino acid residues are modified compared to the reference sequence Seq ID No. 2 by deletion, insertion, substitution or a combination thereof and which still possesses at least 10% of the enzymatic activity of the enzyme with the reference sequence Seq ID No. 2, wherein enzymatic activity for an enzyme E₁ is understood to mean the ability to convert phytosphingosine to triacetylphytosphingosine by transfer of the acetyl residues from three molecules of acetyl coenzyme A,
and the enzyme E₂ is selected from
an enzyme E₂ with polypeptide sequence Seq ID No. 4 or with a polypeptide sequence in which up to 25% of the amino acid residues are modified compared to the reference sequence Seq ID No. 4 by deletion, insertion, substitution or a combination thereof and which still possesses at least 10% of the enzymatic activity of the enzyme with the reference sequence Seq ID No. 4, wherein enzymatic activity for an enzyme E₂ is understood to mean the ability to convert triacetylphytosphingosine to tetraacetylphytosphingosine by transfer of the acetyl residue from one molecule of acetyl coenzyme A,
wherein the increase in the enzymatic activity is achieved by an increased copy number of the gene sequence which codes for the enzyme.

4. Cell according to Claim 3, **characterized in that** it is selected from *Saccharomyces cerevisiae, Kluyveromyces lactis, Hansenula polymorpha, Pichia pastoris, Pichia ciferrii, Yarrowia lipolytica, Candida albicans, Candida utilis* and *Ashbya gossypii.*

5. Use of the cells according to Claim 3 or 4 for the production of acetylated sphingoid bases.

6. Method for the production of acetylated sphingoid bases comprising the process steps
a) contacting the cell according to Claim 3 or 4 with a medium containing a carbon source,
b) culturing the cell under conditions which enable the cell to form sphingoid bases from the carbon source and
c) optionally isolation of the acetylated sphingoid bases formed.

7. Method for the production of N-acetylated, primary aliphatic amines comprising the process step
A) contacting at least one of the enzymes E₁ or E₂, wherein the enzyme E₁ is selected from
an enzyme E₁ with the polypeptide sequence Seq ID No. 2 or with a polypeptide sequence in which up to 25% of the amino acid residues are modified compared to the reference sequence Seq ID No. 2 by deletion, insertion, substitution or a combination thereof and which still possesses at least 10% of the enzymatic activity of the enzyme with the reference sequence Seq ID No. 2, wherein enzymatic activity for an enzyme E₁ is understood to mean the ability to convert phytosphingosine to triacetylphytosphingosine by transfer of the acetyl residues from three molecules of acetyl coenzyme A,
and the enzyme E₂ is selected from
an enzyme E₂ with the polypeptide sequence Seq ID No. 4 or with a polypeptide sequence in which up to 25% of the amino acid residues are modified compared to the reference sequence Seq ID No. 4 by deletion, insertion, substitution or a combination thereof and which still possesses at least 10% of the enzymatic activity of the enzyme with the reference sequence Seq ID No. 4, wherein enzymatic activity for an enzyme E₂ is understood to mean the ability to convert triacetylphytosphingosine to tetraacetylphytosphingosine by transfer of the acetyl residue from one molecule of acetyl coenzyme A,
with a medium containing a primary aliphatic amine,
and acetyl CoA, **characterized in that** cells according to Claim 3 are used as the at least one of the enzymes E1 or E2.

8. Method according to Claim 7, **characterized in that** the primary aliphatic amine is selected from those which have 6 to 18 carbon atoms.

9. Method according to Claim 7 or 8, **characterized in that** it comprises the process step
B) isolation of the acetylated amines formed.

## Revendications

1. Acide nucléique isolé qui présente une séquence choisie parmi les groupes A1) à D1 et G1) :
A1) une séquence selon SED ID No 1, cette séquence codant pour une protéine qui est en mesure de transformer la phytosphingosine par transfert des radicaux acétyle de trois molécules d'acétyl-coenzyme A en triacétylphytosphingosine,
B1) une séquence sans intron, qui est dérivée d'une séquence selon A1) et qui code pour la même protéine ou le même peptide que la séquence selon SEQ ID No 1,
C1) une séquence qui code pour une protéine ou un peptide qui comprend la séquence d'acides aminés selon SEQ ID No 2 et qui est en mesure de transformer la phytosphingosine par transfert des radicaux acétyle de trois molécules d'acétyl-coenzyme A en triacétylphytosphingosine,
D1) une séquence qui est au moins 70 % identique à une séquence selon l'un quelconque des groupes A1) à C1), cette séquence codant pour une protéine ou un peptide qui est en mesure de transformer la phytosphingosine par transfert des radicaux acétyle de trois molécules d'acétyl-coenzyme A en triacétylphytosphingosine,
G1) une séquence complémentaire d'une séquence selon l'un quelconque des groupes A1) à D1).

2. Acide nucléique isolé qui présente une séquence choisie parmi les groupes A2) à D2 et G2) :
A2) une séquence selon SED ID No 3, cette séquence codant pour une protéine qui est en mesure de transformer la triacétylphytosphingosine par transfert du radical acétyle d'une molécule d'acétyl-coenzyme A en tétraacétylphytosphingosine,
B2) une séquence sans intron, qui est dérivée d'une séquence selon A2) et qui code pour la même protéine ou le même peptide que la séquence selon SEQ ID No 3,
C2) une séquence qui code pour une protéine ou un peptide qui comprend la séquence d'acides aminés selon SEQ ID No 4 et qui est en mesure de transformer la triacétylphytosphingosine par transfert du radical acétyle d'une molécule d'acétyl-coenzyme A en tétraacétylphytosphingosine,
D2) une séquence qui est au moins 70 % identique à une séquence selon l'un quelconque des groupes A2) à C2), cette séquence codant pour une protéine ou un peptide qui est en mesure de transformer la triacétylphytosphingosine par transfert du radical acétyle d'une molécule d'acétylcoenzyme A en tétraacétylphytosphingosine,
G2) une séquence complémentaire d'une séquence selon l'un quelconque des groupes A2) à D2).

3. Cellule **caractérisée en ce qu'**elle a été modifiée par génie génétique de sorte qu'elle présente en comparaison de son type sauvage une activité augmentée d'au moins une des enzymes E₁ et/ou E₂, l'enzyme E₁ étant choisie parmi une enzyme E₁ ayant la séquence polypeptidique SEQ ID No 2 ou ayant une séquence polypeptidique dans laquelle jusqu'à 25 % des radicaux acide aminé sont modifiés par rapport à la séquence de référence SEQ ID No 2 par délétion, insertion, substitution ou une combinaison de celles-ci, et qui présente encore au moins 10 % de l'activité enzymatique de l'enzyme ayant la séquence de référence SEQ ID No 2, l'activité enzymatique pour une enzyme E₁ se rapportant à la capacité à transformer la phytosphingosine par transfert des radicaux acétyle de trois molécules d'acétyl-coenzyme A en triacétylphytosphingosine, et l'enzyme E₂ étant choisie parmi une enzyme E₂ ayant la séquence polypeptidique SEQ ID No 4 ou ayant une séquence polypeptidique dans laquelle jusqu'à 25 % des radicaux acide aminé sont modifiés par rapport à la séquence de référence SEQ ID No 4 par délétion, insertion, substitution ou une combinaison de celles-ci, et qui présente encore au moins 10 % de l'activité enzymatique de l'enzyme ayant la séquence de référence SEQ ID No 4, l'activité enzymatique pour une enzyme E₂ se rapportant à la capacité à transformer la triacétylphytosphingosine par transfert du radical acétyle d'une molécule d'acétylcoenzyme A en tétraacétylphytosphingosine, l'augmentation de l'activité enzymatique étant obtenue par un nombre de copies augmenté de la séquence génétique qui code pour l'enzyme.

4. Cellule selon la revendication 3, **caractérisée en ce qu'**elle est choisie parmi *Saccharomyces cerevisiae, Kluyveromyces lactis, Hansenula polymorpha, Pichia pastoris, Pichia ciferrii, Yarrowia lipolytica, Candida albicans, Candida utilis* et *Ashbya gossypii.*

5. Utilisation des cellules selon la revendication 3 ou 4 pour la fabrication de bases sphingoïdes acétylées.

6. Procédé de fabrication de bases sphingoïdes acétylées comprenant les étapes de procédé suivantes:
a) la mise en contact de la cellule selon la revendication 3 ou 4 avec un milieu contenant une source de carbone,
b) la culture de la cellule dans des conditions qui permettent à la cellule de former des bases sphingoïdes à partir de la source de carbone, et
c) éventuellement l'isolement des bases sphingoïdes acétylées formées.

7. Procédé de fabrication d'amines aliphatiques primaires N-acétylées comprenant l'étape de procédé suivante :
A) la mise en contact d'au moins une des enzymes E₁ ou E₂, l'enzyme E₁ étant choisie parmi une enzyme E₁ ayant la séquence polypeptidique SEQ ID No 2 ou ayant une séquence polypeptidique dans laquelle jusqu'à 25 % des radicaux acide aminé sont modifiés par rapport à la séquence de référence SEQ ID No 2 par délétion, insertion, substitution ou une combinaison de celles-ci, et qui présente encore au moins 10 % de l'activité enzymatique de l'enzyme ayant la séquence de référence SEQ ID No 2, l'activité enzymatique pour une enzyme E₁ se rapportant à la capacité à transformer la phytosphingosine par transfert des radicaux acétyle de trois molécules d'acétyl-coenzyme A en triacétylphytosphingosine,
et l'enzyme E₂ étant choisie parmi une enzyme E₂ ayant la séquence polypeptidique SEQ ID No 4 ou ayant une séquence polypeptidique dans laquelle jusqu'à 25 % des radicaux acide aminé sont modifiés par rapport à la séquence de référence SEQ ID No 4 par délétion, insertion, substitution ou une combinaison de celles-ci, et qui présente encore au moins 10 % de l'activité enzymatique de l'enzyme ayant la séquence de référence SEQ ID No 4, l'activité enzymatique pour une enzyme E₂ se rapportant à la capacité à transformer la triacétylphytosphingosine par transfert du radical acétyle d'une molécule d'acétylcoenzyme A en tétraacétylphytosphingosine,
avec un milieu contenant une amine aliphatique primaire et de l'acétyl-CoA,
**caractérisé en ce que** des cellules selon la revendication 3 sont utilisées en tant que ladite au moins une des enzymes E₁ ou E₂.

8. Procédé selon la revendication 7, **caractérisé en ce que** l'amine aliphatique primaire est choisie parmi celles comprenant 6 à 18 atomes de carbone.

9. Procédé selon la revendication 7 ou 8, **caractérisé en ce qu'**il comprend l'étape de procédé suivante :
B) l'isolement des amines acétylées formées.


### SEQUENCE LISTING

<110> Evonik Industries
<120> Neue Enzyme
<130> 201100515
<160> 12
<170> PatentIn version 3.5
<210> 1
   <211> 1326
   <212> DNA
   <213> Pichia ciferrii
<220>
   <221> CDS
   <222> (1)..(1326)
<400> 1
   atg gtg gct gga cca aac aaa gat ctt gaa aac ctg gaa cgt atg atg          48
   Met Val Ala Gly Pro Asn Lys Asp Leu Glu Asn Leu Glu Arg Met Met
   1               5                   10                  15
   tac tgg aag acc act ttg aaa gct tgg tca tgt ttc ctt gtt ggt gct          96
   Tyr Trp Lys Thr Thr Leu Lys Ala Trp Ser Cys Phe Leu Val Gly Ala
               20                  25                  30
   aaa tta aac gaa aaa tta gaa aca gat gat att tta aaa ggt atc cac         144
   Lys Leu Asn Glu Lys Leu Glu Thr Asp Asp Ile Leu Lys Gly Ile His
           35                  40                  45
   aaa tta ttc acg ttg agg gtt cag tta cgt ttg aat gtt ttc caa tat         192
   Lys Leu Phe Thr Leu Arg Val Gln Leu Arg Leu Asn Val Phe Gln Tyr
       50                  55                  60
   cct aaa aaa agg ttt gtt acc gaa gag ata aat ggt tgg tct gat gat         240
   Pro Lys Lys Arg Phe Val Thr Glu Glu Ile Asn Gly Trp Ser Asp Asp
   65                  70                  75                  80
   ttt gtt gat ttt gtc gat tat cca act gat gat ttt gat att att gaa         288
   Phe Val Asp Phe Val Asp Tyr Pro Thr Asp Asp Phe Asp Ile Ile Glu
                   85                  90                  95
   gct ttt aaa caa caa cat aat caa tat ttt gaa ttg ggt gtt caa aag         336
   Ala Phe Lys Gln Gln His Asn Gln Tyr Phe Glu Leu Gly Val Gln Lys
               100                 105                 110
   cct tta tgg aaa ttg gtt gta ttg aac cat caa tat tta gtt att ctt         384
   Pro Leu Trp Lys Leu Val Val Leu Asn His Gln Tyr Leu Val Ile Leu
           115                 120                 125
   tgt gat cat acc tta tat gat ggg aac act gca ctt tat ata tgt gag         432
   Cys Asp His Thr Leu Tyr Asp Gly Asn Thr Ala Leu Tyr Ile Cys Glu
       130                 135                 140
   gat ttg atc aca ata ttg aat gat cgt gat atc cca gtt gat aga att         480
   Asp Leu Ile Thr Ile Leu Asn Asp Arg Asp Ile Pro Val Asp Arg Ile
   145                 150                 155                 160
   cca gat att aaa cca tat cat gat cta tta aaa cca aaa ctt gga cat         528
   Pro Asp Ile Lys Pro Tyr His Asp Leu Leu Lys Pro Lys Leu Gly His
                   165                 170                 175
   aca atc aaa act gtc atc caa act ttt gca cca aaa tgg gct tat cct         576
   Thr Ile Lys Thr Val Ile Gln Thr Phe Ala Pro Lys Trp Ala Tyr Pro
               180                 185                 190
   tta gtt aat ctg att tat aga cca aaa agt gaa ttt gaa act ggt gca         624
   Leu Val Asn Leu Ile Tyr Arg Pro Lys Ser Glu Phe Glu Thr Gly Ala
           195                 200                 205
   tat gat gat tgg gga gta act cat aaa att gaa aga aca aca aat aaa         672
   Tyr Asp Asp Trp Gly Val Thr His Lys Ile Glu Arg Thr Thr Asn Lys
       210                 215                 220
   tta aag cac tta att aca ata act aat gaa gaa ttt tcc ata att aaa         720
   Leu Lys His Leu Ile Thr Ile Thr Asn Glu Glu Phe Ser Ile Ile Lys
   225                 230                 235                 240
   aaa tta aca aaa tca cat ggt gta aat ttc aca gca ttt tgg gca tat         768
   Lys Leu Thr Lys Ser His Gly Val Asn Phe Thr Ala Phe Trp Ala Tyr
                   245                 250                 255
   atc aat gtt ctt gca gtt gca caa ttg gga aag tca gct gtt gat tta         816
   Ile Asn Val Leu Ala Val Ala Gln Leu Gly Lys Ser Ala Val Asp Leu
               260                 265                 270
   tca att cca ttc aat atg aga acc aat tta tta cca cca gaa tat tta         864
   Ser Ile Pro Phe Asn Met Arg Thr Asn Leu Leu Pro Pro Glu Tyr Leu
           275                 280                 285
   aga tgg tat ggt tta tta gtt tca cat gtt act tta aat gta cat acc         912
   Arg Trp Tyr Gly Leu Leu Val Ser His Val Thr Leu Asn Val His Thr
       290                 295                 300
   aaa gtt gat cat gat tca att gac tgg gat ttt gtt aga ttt tta aat         960
   Lys Val Asp His Asp Ser Ile Asp Trp Asp Phe Val Arg Phe Leu Asn
   305                 310                 315                 320
   ggt agt gtt gca cat aaa tac caa gta aaa caa tca caa atg ctt gga        1008
   Gly Ser Val Ala His Lys Tyr Gln Val Lys Gln Ser Gln Met Leu Gly
                   325                 330                 335
   atg att aaa tat gtt agt gct cgt gga ctt att gaa tca gct tta aaa        1056
   Met Ile Lys Tyr Val Ser Ala Arg Gly Leu Ile Glu Ser Ala Leu Lys
               340                 345                 350
   tca cca aga aaa ggt gga tta gaa gtt tca aac ttg gga ttg aga gtc        1104
   Ser Pro Arg Lys Gly Gly Leu Glu Val Ser Asn Leu Gly Leu Arg Val
           355                 360                 365
   gat cca gat ggt gaa tca tgg aaa aaa tat acc cct gaa gaa ttt ttc        1152
   Asp Pro Asp Gly Glu Ser Trp Lys Lys Tyr Thr Pro Glu Glu Phe Phe
       370                 375                 380
   ttt tct ttg cca aat gat ctt tca ggt tat aat gtt tca aat gct gtg        1200
   Phe Ser Leu Pro Asn Asp Leu Ser Gly Tyr Asn Val Ser Asn Ala Val
   385                 390                 395                 400
   att tca agt aaa act aaa aca aat att att tta gac ggt gtt cca gaa        1248
   Ile Ser Ser Lys Thr Lys Thr Asn Ile Ile Leu Asp Gly Val Pro Glu
                   405                 410                 415
   ttt gca aat gaa ttt cca acg tat gca aat aac gtt gaa aca att ttg       1296
   Phe Ala Asn Glu Phe Pro Thr Tyr Ala Asn Asn Val Glu Thr Ile Leu
               420                 425                 430
   aga aat gca atc aat ggg tat tat gaa taa                               1326
   Arg Asn Ala Ile Asn Gly Tyr Tyr Glu
           435                 440
<210> 2
   <211> 441
   <212> PRT
   <213> Pichia ciferrii
<400> 2
   Met Val Ala Gly Pro Asn Lys Asp Leu Glu Asn Leu Glu Arg Met Met
   1               5                   10                  15
   Tyr Trp Lys Thr Thr Leu Lys Ala Trp Ser Cys Phe Leu Val Gly Ala
               20                  25                  30
   Lys Leu Asn Glu Lys Leu Glu Thr Asp Asp Ile Leu Lys Gly Ile His
           35                  40                  45
   Lys Leu Phe Thr Leu Arg Val Gln Leu Arg Leu Asn Val Phe Gln Tyr
       50                  55                  60
   Pro Lys Lys Arg Phe Val Thr Glu Glu Ile Asn Gly Trp Ser Asp Asp
   65                  70                  75                  80
   Phe Val Asp Phe Val Asp Tyr Pro Thr Asp Asp Phe Asp Ile Ile Glu
                   85                  90                  95
   Ala Phe Lys Gln Gln His Asn Gln Tyr Phe Glu Leu Gly Val Gln Lys
               100                 105                 110
   Pro Leu Trp Lys Leu Val Val Leu Asn His Gln Tyr Leu Val Ile Leu
           115                 120                 125
   Cys Asp His Thr Leu Tyr Asp Gly Asn Thr Ala Leu Tyr Ile Cys Glu
       130                 135                 140
   Asp Leu Ile Thr Ile Leu Asn Asp Arg Asp Ile Pro Val Asp Arg Ile
   145                 150                 155                 160
   Pro Asp Ile Lys Pro Tyr His Asp Leu Leu Lys Pro Lys Leu Gly His
                   165                 170                 175
   Thr Ile Lys Thr Val Ile Gln Thr Phe Ala Pro Lys Trp Ala Tyr Pro
               180                 185                 190
   Leu Val Asn Leu Ile Tyr Arg Pro Lys Ser Glu Phe Glu Thr Gly Ala
           195                 200                 205
   Tyr Asp Asp Trp Gly Val Thr His Lys Ile Glu Arg Thr Thr Asn Lys
       210                 215                 220
   Leu Lys His Leu Ile Thr Ile Thr Asn Glu Glu Phe Ser Ile Ile Lys
   225                 230                 235                 240
   Lys Leu Thr Lys Ser His Gly Val Asn Phe Thr Ala Phe Trp Ala Tyr
                   245                 250                 255
   Ile Asn Val Leu Ala Val Ala Gln Leu Gly Lys Ser Ala Val Asp Leu
               260                 265                 270
   Ser Ile Pro Phe Asn Met Arg Thr Asn Leu Leu Pro Pro Glu Tyr Leu
           275                 280                 285
   Arg Trp Tyr Gly Leu Leu Val Ser His Val Thr Leu Asn Val His Thr
       290                 295                 300
   Lys Val Asp His Asp Ser Ile Asp Trp Asp Phe Val Arg Phe Leu Asn
   305                 310                 315                 320
   Gly Ser Val Ala His Lys Tyr Gln Val Lys Gln Ser Gln Met Leu Gly
                   325                 330                 335
   Met Ile Lys Tyr Val Ser Ala Arg Gly Leu Ile Glu Ser Ala Leu Lys
               340                 345                 350
   Ser Pro Arg Lys Gly Gly Leu Glu Val Ser Asn Leu Gly Leu Arg Val
           355                 360                 365
   Asp Pro Asp Gly Glu Ser Trp Lys Lys Tyr Thr Pro Glu Glu Phe Phe
       370                 375                 380
   Phe Ser Leu Pro Asn Asp Leu Ser Gly Tyr Asn Val Ser Asn Ala Val
   385                 390                 395                 400
   Ile Ser Ser Lys Thr Lys Thr Asn Ile Ile Leu Asp Gly Val Pro Glu
                   405                 410                 415
   Phe Ala Asn Glu Phe Pro Thr Tyr Ala Asn Asn Val Glu Thr Ile Leu
               420                 425                 430
   Arg Asn Ala Ile Asn Gly Tyr Tyr Glu
   435 440
<210> 3
   <211> 1431
   <212> DNA
   <213> Pichia ciferrii
<220>
   <221> CDS
   <222> (1)..(1431)
<400> 3
   atg tca ttt aaa tat atc aat caa aat gat tca aaa tca tta tca aat        48
   Met Ser Phe Lys Tyr Ile Asn Gln Asn Asp Ser Lys Ser Leu Ser Asn
   1               5                   10                  15
   tta aaa tat aaa tta tca aaa aat cat gca aga caa atg ggt ttt tta        96
   Leu Lys Tyr Lys Leu Ser Lys Asn His Ala Arg Gln Met Gly Phe Leu
               20                  25                  30
   gaa gat ttt ttt gca att tta caa cgt caa aaa atg tat aaa tca ttt       144
   Glu Asp Phe Phe Ala Ile Leu Gln Arg Gln Lys Met Tyr Lys Ser Phe
           35                  40                  45
   ttc gtt atg tgt aaa tat aat gaa aaa att gat gat ttt aaa att tta       192
   Phe Val Met Cys Lys Tyr Asn Glu Lys Ile Asp Asp Phe Lys Ile Leu
       50                  55                  60
   ttc cat tca tta aga tta tta ata tta aaa ttc cca ata tta gct tcc       240
   Phe His Ser Leu Arg Leu Leu Ile Leu Lys Phe Pro Ile Leu Ala Ser
   65                  70                  75                  80
   aca ata att act caa aat gtt cca att aat ata aaa cct cgt cct tat       288
   Thr Ile Ile Thr Gln Asn Val Pro Ile Asn Ile Lys Pro Arg Pro Tyr
                   85                  90                  95
   gat tat att caa att att gat gaa ata aaa ttt aat gat ttg gtt tgg       336
   Asp Tyr Ile Gln Ile Ile Asp Glu Ile Lys Phe Asn Asp Leu Val Trp
               100                 105                 110
   gat tta aga cct gaa tat tca aat tta tta caa gaa gat tta tta aat       384
   Asp Leu Arg Pro Glu Tyr Ser Asn Leu Leu Gln Glu Asp Leu Leu Asn
           115                 120                 125
   aaa tta aat gat tta att ata cca tat gaa gat aat aaa tta gtt tgg       432
   Lys Leu Asn Asp Leu Ile Ile Pro Tyr Glu Asp Asn Lys Leu Val Trp
       130                 135                 140
   aga tta gga atc ttg gat gat tat aca tta att ttt ata aca aat cat       480
   Arg Leu Gly Ile Leu Asp Asp Tyr Thr Leu Ile Phe Ile Thr Asn His
   145                 150                 155                 160
   gtt tta cat gat gga ata tct ggt aaa aat att ttt aat gaa tta tca       528
   Val Leu His Asp Gly Ile Ser Gly Lys Asn Ile Phe Asn Glu Leu Ser
                   165                 170                 175
   tta att ttt aat caa ttg gac ttg gat tct tta agt gat gat gat gat       576
   Leu Ile Phe Asn Gln Leu Asp Leu Asp Ser Leu Ser Asp Asp Asp Asp
               180                 185                 190
   atc gtg ttc aat tat tca caa gat cat ttg aat tta ggt gaa tta cca       624
   Ile Val Phe Asn Tyr Ser Gln Asp His Leu Asn Leu Gly Glu Leu Pro
           195                 200                 205
   aaa cct ata act gat ctt atg aat cat att cca tca att aaa tct tta        672
   Lys Pro Ile Thr Asp Leu Met Asn His Ile Pro Ser Ile Lys Ser Leu
       210                 215                 220
   cca aga tat att tat aat tca tta att gaa cca aaa ctt ttt tgt tca        720
   Pro Arg Tyr Ile Tyr Asn Ser Leu Ile Glu Pro Lys Leu Phe Cys Ser
   225                 230                 235                 240
   tca act tta att caa ggt cat ctt aag aat att cat tat aga gtt aat        768
   Ser Thr Leu Ile Gln Gly His Leu Lys Asn Ile His Tyr Arg Val Asn
                   245                 250                 255
   ata aat cca atg gaa tta tta aaa att aaa tca tta tta tca aaa aat        816
   Ile Asn Pro Met Glu Leu Leu Lys Ile Lys Ser Leu Leu Ser Lys Asn
               260                 265                 270
   agt ttc aat aat gtt aaa tta act tta aca cct ttc att caa tct att        864
   Ser Phe Asn Asn Val Lys Leu Thr Leu Thr Pro Phe Ile Gln Ser Ile
           275                 280                 285
   tgg aat tat act tta tat caa gat gaa tat tat aaa tca tca aaa tct        912
   Trp Asn Tyr Thr Leu Tyr Gln Asp Glu Tyr Tyr Lys Ser Ser Lys Ser
       290                 295                 300
   tta tta ggt att gca gtg gat tct cgt caa ttt att aat aaa gat gaa        960
   Leu Leu Gly Ile Ala Val Asp Ser Arg Gln Phe Ile Asn Lys Asp Glu
   305                 310                 315                 320
   caa gat tta tat aaa ttt ggt tta aat gta tca ggt ttt agt aaa att       1008
   Gln Asp Leu Tyr Lys Phe Gly Leu Asn Val Ser Gly Phe Ser Lys Ile
                   325                 330                 335
   tcc aaa cca atg aaa tta att aca tgg aat aaa att aat caa att aat       1056
   Ser Lys Pro Met Lys Leu Ile Thr Trp Asn Lys Ile Asn Gln Ile Asn
               340                 345                 350
   caa gat tta aaa att tca tta aaa ttg aaa aaa cct tta tat tca atg       1104
   Gln Asp Leu Lys Ile Ser Leu Lys Leu Lys Lys Pro Leu Tyr Ser Met
           355                 360                 365
   ggt ata tta ggt tgg gat aaa atg att aaa aat aaa cat tta gat gtt       1152
   Gly Ile Leu Gly Trp Asp Lys Met Ile Lys Asn Lys His Leu Asp Val
       370                 375                 380
   gat tta cca aaa att atg aat aaa aga aca ggt tca act ttt tca aat       1200
   Asp Leu Pro Lys Ile Met Asn Lys Arg Thr Gly Ser Thr Phe Ser Asn
   385                 390                 395                 400
   att ggt ata atc cta aat aac agt gaa tca aat gat aaa ttt caa att       1248
   Ile Gly Ile Ile Leu Asn Asn Ser Glu Ser Asn Asp Lys Phe Gln Ile
                   405                 410                 415
   att gat gca atg ttt aca caa cat ttt aat gtt cat ttt tat gat ttc       1296
   Ile Asp Ala Met Phe Thr Gln His Phe Asn Val His Phe Tyr Asp Phe
               420                 425                 430
   tca atc act gca att tct aca atg act ggt ggg tta aat att ata att       1344
   Ser Ile Thr Ala Ile Ser Thr Met Thr Gly Gly Leu Asn Ile Ile Ile
           435                 440                 445
   aca tca cca gaa tct att gga att gaa aat tta gaa aga att tgt aaa       1392
   Thr Ser Pro Glu Ser Ile Gly Ile Glu Asn Leu Glu Arg Ile Cys Lys
       450                 455                 460
   aaa ttt cat gaa aat tta gtt tta tgt gat att aaa taa                   1431
   Lys Phe His Glu Asn Leu Val Leu Cys Asp Ile Lys
   465                 470                 475
<210> 4
   <211> 476
   <212> PRT
   <213> Pichia ciferrii
<400> 4
   Met Ser Phe Lys Tyr Ile Asn Gln Asn Asp Ser Lys Ser Leu Ser Asn
   1               5                   10                  15
   Leu Lys Tyr Lys Leu Ser Lys Asn His Ala Arg Gln Met Gly Phe Leu
               20                  25                  30
   Glu Asp Phe Phe Ala Ile Leu Gln Arg Gln Lys Met Tyr Lys Ser Phe
           35                  40                  45
   Phe Val Met Cys Lys Tyr Asn Glu Lys Ile Asp Asp Phe Lys Ile Leu
       50                  55                  60
   Phe His Ser Leu Arg Leu Leu Ile Leu Lys Phe Pro Ile Leu Ala Ser
   65                  70                  75                  80
   Thr Ile Ile Thr Gln Asn Val Pro Ile Asn Ile Lys Pro Arg Pro Tyr
                   85                  90                  95
   Asp Tyr Ile Gln Ile Ile Asp Glu Ile Lys Phe Asn Asp Leu Val Trp
               100                 105                 110
   Asp Leu Arg Pro Glu Tyr Ser Asn Leu Leu Gln Glu Asp Leu Leu Asn
           115                 120                 125
   Lys Leu Asn Asp Leu Ile Ile Pro Tyr Glu Asp Asn Lys Leu Val Trp
       130                 135                 140
   Arg Leu Gly Ile Leu Asp Asp Tyr Thr Leu Ile Phe Ile Thr Asn His
   145                 150                 155                 160
   Val Leu His Asp Gly Ile Ser Gly Lys Asn Ile Phe Asn Glu Leu Ser
                   165                 170                 175
   Leu Ile Phe Asn Gln Leu Asp Leu Asp Ser Leu Ser Asp Asp Asp Asp
               180                 185                 190
   Ile Val Phe Asn Tyr Ser Gln Asp His Leu Asn Leu Gly Glu Leu Pro
           195                 200                 205
   Lys Pro Ile Thr Asp Leu Met Asn His Ile Pro Ser Ile Lys Ser Leu
       210                 215                 220
   Pro Arg Tyr Ile Tyr Asn Ser Leu Ile Glu Pro Lys Leu Phe Cys Ser
   225                 230                 235                 240
   Ser Thr Leu Ile Gln Gly His Leu Lys Asn Ile His Tyr Arg Val Asn
                   245                 250                 255
   Ile Asn Pro Met Glu Leu Leu Lys Ile Lys Ser Leu Leu Ser Lys Asn
               260                 265                 270
   Ser Phe Asn Asn Val Lys Leu Thr Leu Thr Pro Phe Ile Gln Ser Ile
           275                 280                 285
   Trp Asn Tyr Thr Leu Tyr Gln Asp Glu Tyr Tyr Lys Ser Ser Lys Ser
       290                 295                 300
   Leu Leu Gly Ile Ala Val Asp Ser Arg Gln Phe Ile Asn Lys Asp Glu
   305                 310                 315                 320
   Gln Asp Leu Tyr Lys Phe Gly Leu Asn Val Ser Gly Phe Ser Lys Ile
                   325                 330                 335
   Ser Lys Pro Met Lys Leu Ile Thr Trp Asn Lys Ile Asn Gln Ile Asn
               340                 345                 350
   Gln Asp Leu Lys Ile Ser Leu Lys Leu Lys Lys Pro Leu Tyr Ser Met
           355                 360                 365
   Gly Ile Leu Gly Trp Asp Lys Met Ile Lys Asn Lys His Leu Asp Val
       370                 375                 380
   Asp Leu Pro Lys Ile Met Asn Lys Arg Thr Gly Ser Thr Phe Ser Asn
   385                 390                 395                 400
   Ile Gly Ile Ile Leu Asn Asn Ser Glu Ser Asn Asp Lys Phe Gln Ile
                   405                 410                 415
   Ile Asp Ala Met Phe Thr Gln His Phe Asn Val His Phe Tyr Asp Phe
               420                 425                 430
   Ser Ile Thr Ala Ile Ser Thr Met Thr Gly Gly Leu Asn Ile Ile Ile
           435                 440                 445
   Thr Ser Pro Glu Ser Ile Gly Ile Glu Asn Leu Glu Arg Ile Cys Lys
       450                 455                 460
   Lys Phe His Glu Asn Leu Val Leu Cys Asp Ile Lys
   465                 470                 475
<210> 5
   <211> 7483
   <212> DNA
   <213> Artificial Sequence
<220>
   <223> Vector
<400> 5
   cgtcaggggg gcggagccta tggaaaaacg ccagcaacgc ggccttttta cggttcctgg      60
   ccttttgctg gccttttgct cacatgttct ttcctgcgtt atcccctgat tctgtggata     120
   accgtattac cgcctttgag tgagctgata ccgctcgccg cagccgaacg accgagcgca     180
   gcgagtcagt gagcgaggaa gcggaagagc gcccaatacg caaaccgcct ctccccgcgc     240
   gttggccgat tcattaatgc agctggcacg acaggtttcc cgactggaaa gcgggcagtg     300
   agcgcaacgc aattaatgtg agttacctca ctcattaggc accccaggct ttacacttta     360
   tgcttccggc tcctatgttg tgtggaattg tgagcggata acaatttcac acaggaaaca     420
   gctatgacca tgattacgcc aagcgcgcaa ttaaccctca ctaaagggaa caaaagctgg     480
   agctcgtagg aacaatttcg ggcccctgcg tgttcttctg aggttcatct tttacatttg     540
   cttctgctgg ataattttca gaggcaacaa ggaaaaatta gatggcaaaa agtcgtcttt     600
   caaggaaaaa tccccaccat ctttcgagat cccctgtaac ttattggcaa ctgaaagaat     660
   gaaaaggagg aaaatacaaa atatactaga actgaaaaaa aaaaagtata aatagagacg     720
   atatatgcca atacttcaca atgttcgaat ctattcttca tttgcagcta ttgtaaaata     780
   ataaaacatc aagaacaaac aagctcaact tgtcttttct aagaacaaag aataaacaca     840
   aaaacaaaaa gtttttttaa ttttaatcaa aaagttaaca tgcatcacca tcaccatcac     900
   actagtggat cccccgggct gcaggaattc gatatcaagc ttatcgatac cgtcgacctc     960
   gagtcatgta attagttatg tcacgcttac attcacgccc tccccccaca tccgctctaa    1020
   ccgaaaagga aggagttaga caacctgaag tctaggtccc tatttatttt tttatagtta    1080
   tgttagtatt aagaacgtta tttatatttc aaatttttct tttttttctg tacagacgcg    1140
   tgtacgcatg taacattata ctgaaaacct tgcttgagaa ggttttggga cgctcgaagg    1200
   ctttaatttg cggccggtac ccaattcgcc ctatagtgag tcgtattacg cgcgctcact    1260
   ggccgtcgtt ttacaacgtc gtgactggga aaaccctggc gttacccaac ttaatcgcct    1320
   tgcagcacat ccccctttcg ccagctggcg taatagcgaa gaggcccgca ccgatcgccc 1380
   ttcccaacag ttgcgcagcc tgaatggcga atggcgcgac gcgccctgta gcggcgcatt 1440
   aagcgcggcg ggtgtggtgg ttacgcgcag cgtgaccgct acacttgcca gcgccctagc 1500
   gcccgctcct ttcgctttct tcccttcctt tctcgccacg ttcgccggct ttccccgtca 1560
   agctctaaat cgggggctcc ctttagggtt ccgatttagt gctttacggc acctcgaccc 1620
   caaaaaactt gattagggtg atggttcacg tagtgggcca tcgccctgat agacggtttt 1680
   tcgccctttg acgttggagt ccacgttctt taatagtgga ctcttgttcc aaactggaac 1740
   aacactcaac cctatctcgg tctattcttt tgatttataa gggattttgc cgatttcggc 1800
   ctattggtta aaaaatgagc tgatttaaca aaaatttaac gcgaatttta acaaaatatt 1860
   aacgtttaca atttcctgat gcggtatttt ctccttacgc atctgtgcgg tatttcacac 1920
   cgcatatcga cggtcgagga gaacttctag tatatccaca tacctaatat tattgcctta 1980
   ttaaaaatgg aatcccaaca attacatcaa aatccacatt ctcttcaaaa tcaattgtcc 2040
   tgtacttcct tgttcatgtg tgttcaaaaa cgttatattt ataggataat tatactctat 2100
   ttctcaacaa gtaattggtt gtttggccga gcggtctaag gcgcctgatt caagaaatat 2160
   cttgaccgca gttaactgtg ggaatactca ggtatcgtaa gatgcaagag ttcgaatctc 2220
   ttagcaacca ttattttttt cctcaacata acgagaacac acaggggcgc tatcgcacag 2280
   aatcaaattc gatgactgga atttttttgt taatttcaga ggtcgcctga cgcatatacc 2340
   tttttcaact gaaaaattgg gagaaaaagg aaaggtgaga ggccggaacc ggcttttcat 2400
   atagaataga gaagcgttca tgactaaatg cttgcatcac aatacttgaa gttgacaata 2460
   ttatttaagg acctattgtt ttttccaata ggtggttagc aatcgtctta ctttctaact 2520
   tttcttacct tttacatttc agcaatatat atatatattt caaggatata ccattctaat 2580
   gtctgcccct atgtctgccc ctaagaagat cgtcgttttg ccaggtgacc acgttggtca 2640
   agaaatcaca gccgaagcca ttaaggttct taaagctatt tctgatgttc gttccaatgt 2700
   caagttcgat ttcgaaaatc atttaattgg tggtgctgct atcgatgcta caggtgtccc 2760
   acttccagat gaggcgctgg aagcctccaa gaaggttgat gccgttttgt taggtgctgt 2820
   ggctggtcct aaatggggta ccggtagtgt tagacctgaa caaggtttac taaaaatccg 2880
   taaagaactt caattgtacg ccaacttaag accatgtaac tttgcatccg actctctttt 2940
   agacttatct ccaatcaagc cacaatttgc taaaggtact gacttcgttg ttgtcagaga 3000
   attagtggga ggtatttact ttggtaagag aaaggaagac gatggtgatg gtgtcgcttg 3060
   ggatagtgaa caatacaccg ttccagaagt gcaaagaatc acaagaatgg ccgctttcat 3120
   ggccctacaa catgagccac cattgcctat ttggtccttg gataaagcta atcttttggc 3180
   ctcttcaaga ttatggagaa aaactgtgga ggaaaccatc aagaacgaat tccctacatt 3240
   gaaggttcaa catcaattga ttgattctgc cgccatgatc ctagttaaga acccaaccca     3300
   cctaaatggt attataatca ccagcaacat gtttggtgat atcatctccg atgaagcctc     3360
   cgttatccca ggttccttgg gtttgttgcc atctgcgtcc ttggcctctt tgccagacaa     3420
   gaacaccgca tttggtttgt acgaaccatg ccacggttct gctccagatt tgccaaagaa     3480
   taaggttgac cctatcgcca ctatcttgtc tgctgcaatg atgttgaaat tgtcattgaa     3540
   cttgcctgaa gaaggtaagg ccattgaaga tgcagttaaa aaggttttgg atgcaggtat     3600
   cagaactggt gatttaggtg gttccaacag taccaccgaa gtcggtgatg ctgtcgccga     3660
   agaagttaag aaaatccttg cttaaaaaga ttctcttttt ttatgatatt tgtacataaa     3720
   ctttataaat gaaattcata atagaaacga cacgaaatta caaaatggaa tatgttcata     3780
   gggtagacga aactatatac gcaatctaca tacatttatc aagaaggaga aaaaggagga     3840
   tagtaaagga atacaggtaa gcaaattgat actaatggct caacgtgata aggaaaaaga     3900
   attgcacttt aacattaata ttgacaagga ggagggcacc acacaaaaag ttaggtgtaa     3960
   cagaaaatca tgaaactacg attcctaatt tgatattgga ggattttctc taaaaaaaaa     4020
   aaaatacaac aaataaaaaa cactcaatga cctgaccatt tgatggagtt taagtcaata     4080
   ccttcttgaa gcatttccca taatggtgaa agttccctca agaattttac tctgtcagaa     4140
   acggccttac gacgtagtcg atatggtgca ctctcagtac aatctgctct gatgccgcat     4200
   agttaagcca gccccgacac ccgccaacac ccgctgacgc gccctgacgg gcttgtctgc     4260
   tcccggcatc cgcttacaga caagctgtga ccgtctccgg gagctgcatg tgtcagaggt     4320
   tttcaccgtc atcaccgaaa cgcgcgagac gaaagggcct cgtgatacgc ctatttttat     4380
   aggttaatgt catgataata atggtttctt agtatgatcc aatatcaaag gaaatgatag     4440
   cattgaagga tgagactaat ccaattgagg agtggcagca tatagaacag ctaaagggta     4500
   gtgctgaagg aagcatacga taccccgcat ggaatgggat aatatcacag gaggtactag     4560
   actacctttc atcctacata aatagacgca tataagtacg catttaagca taaacacgca     4620
   ctatgccgtt cttctcatgt atatatatat acaggcaaca cgcagatata ggtgcgacgt     4680
   gaacagtgag ctgtatgtgc gcagctcgcg ttgcattttc ggaagcgctc gttttcggaa     4740
   acgctttgaa gttcctattc cgaagttcct attctctaga aagtatagga acttcagagc     4800
   gcttttgaaa accaaaagcg ctctgaagac gcactttcaa aaaaccaaaa acgcaccgga     4860
   ctgtaacgag ctactaaaat attgcgaata ccgcttccac aaacattgct caaaagtatc     4920
   tctttgctat atatctctgt gctatatccc tatataacct acccatccac ctttcgctcc     4980
   ttgaacttgc atctaaactc gacctctaca ttttttatgt ttatctctag tattactctt     5040
   tagacaaaaa aattgtagta agaactattc atagagtgaa tcgaaaacaa tacgaaaatg     5100
   taaacatttc ctatacgtag tatatagaga caaaatagaa gaaaccgttc ataattttct 5160
   gaccaatgaa gaatcatcaa cgctatcact ttctgttcac aaagtatgcg caatccacat 5220
   cggtatagaa tataatcggg gatgccttta tcttgaaaaa atgcacccgc agcttcgcta 5280
   gtaatcagta aacgcgggaa gtggagtcag gcttttttta tggaagagaa aatagacacc 5340
   aaagtagcct tcttctaacc ttaacggacc tacagtgcaa aaagttatca agagactgca 5400
   ttatagagcg cacaaaggag aaaaaaagta atctaagatg ctttgttaga aaaatagcgc 5460
   tctcgggatg catttttgta gaacaaaaaa gaagtataga ttctttgttg gtaaaatagc 5520
   gctctcgcgt tgcatttctg ttctgtaaaa atgcagctca gattctttgt ttgaaaaatt 5580
   agcgctctcg cgttgcattt ttgttttaca aaaatgaagc acagattctt cgttggtaaa 5640
   atagcgcttt cgcgttgcat ttctgttctg taaaaatgca gctcagattc tttgtttgaa 5700
   aaattagcgc tctcgcgttg catttttgtt ctacaaaatg aagcacagat gcttcgttca 5760
   ggtggcactt ttcggggaaa tgtgcgcgga acccctattt gtttattttt ctaaatacat 5820
   tcaaatatgt atccgctcat gagacaataa ccctgataaa tgcttcaata atattgaaaa 5880
   aggaagagta tgagtattca acatttccgt gtcgccctta ttcccttttt tgcggcattt 5940
   tgccttcctg tttttgctca cccagaaacg ctggtgaaag taaaagatgc tgaagatcag 6000
   ttgggtgcac gagtgggtta catcgaactg gatctcaaca gcggtaagat ccttgagagt 6060
   tttcgccccg aagaacgttt tccaatgatg agcactttta aagttctgct atgtggcgcg 6120
   gtattatccc gtattgacgc cgggcaagag caactcggtc gccgcataca ctattctcag 6180
   aatgacttgg ttgagtactc accagtcaca gaaaagcatc ttacggatgg catgacagta 6240
   agagaattat gcagtgctgc cataaccatg agtgataaca ctgcggccaa cttacttctg 6300
   acaacgatcg gaggaccgaa ggagctaacc gcttttttgc acaacatggg ggatcatgta 6360
   actcgccttg atcgttggga accggagctg aatgaagcca taccaaacga cgagcgtgac 6420
   accacgatgc ctgtagcaat ggcaacaacg ttgcgcaaac tattaactgg cgaactactt 6480
   actctagctt cccggcaaca attaatagac tggatggagg cggataaagt tgcaggacca 6540
   cttctgcgct cggcccttcc ggctggctgg tttattgctg ataaatctgg agccggtgag 6600
   cgtgggtctc gcggtatcat tgcagcactg gggccagatg gtaagccctc ccgtatcgta 6660
   gttatctaca cgacggggag tcaggcaact atggatgaac gaaatagaca gatcgctgag 6720
   ataggtgcct cactgattaa gcattggtaa ctgtcagacc aagtttactc atatatactt 6780
   tagattgatt taaaacttca tttttaattt aaaaggatct aggtgaagat cctttttgat 6840
   aatctcatga ccaaaatccc ttaacgtgag ttttcgttcc actgagcgtc agaccccgta 6900
   gaaaagatca aaggatcttc ttgagatcct ttttttctgc gcgtaatctg ctgcttgcaa 6960
   acaaaaaaac caccgctacc agcggtggtt tgtttgccgg atcaagagct accaactctt 7020
   tttccgaagg taactggctt cagcagagcg cagataccaa atactgtcct tctagtgtag      7080
   ccgtagttag gccaccactt caagaactct gtagcaccgc ctacatacct cgctctgcta      7140
   atcctgttac cagtggctgc tgccagtggc gataagtcgt gtcttaccgg gttggactca      7200
   agacgatagt taccggataa ggcgcagcgg tcgggctgaa cggggggttc gtgcacacag      7260
   cccagcttgg agcgaacgac ctacaccgaa ctgagatacc tacagcgtga gctatgagaa      7320
   agcgccacgc ttcccgaagg gagaaaggcg gacaggtatc cggtaagcgg cagggtcgga      7380
   acaggagagc gcacgaggga gcttccaggg ggaaacgcct ggtatcttta tagtcctgtc      7440
   gggtttcgcc acctctgact tgagcgtcga tttttgtgat gct                         7483
<210> 6
   <211> 6360
   <212> DNA
   <213> Artificial Sequence
<220>
   <223> Vector
<400> 6
   ctctgacttg agcgtcgatt tttgtgatgc tcgtcagggg ggcggagcct atggaaaaac 60
   gccagcaacg cggccttttt acggttcctg gccttttgct ggccttttgc tcacatgttc 120
   tttcctgcgt tatcccctga ttctgtggat aaccgtatta ccgcctttga gtgagctgat 180
   accgctcgcc gcagccgaac gaccgagcgc agcgagtcag tgagcgagga agcggaagag 240
   cgcccaatac gcaaaccgcc tctccccgcg cgttggccga ttcattaatg cagctggcac 300
   gacaggtttc ccgactggaa agcgggcagt gagcgcaacg caattaatgt gagttacctc 360
   actcattagg caccccaggc tttacacttt atgcttccgg ctcctatgtt gtgtggaatt 420
   gtgagcggat aacaatttca cacaggaaac agctatgacc atgattacgc caagcgcgca 480
   attaaccctc actaaaggga acaaaagctg gagctcgtag gaacaatttc gggcccctgc 540
   gtgttcttct gaggttcatc ttttacattt gcttctgctg gataattttc agaggcaaca 600
   aggaaaaatt agatggcaaa aagtcgtctt tcaaggaaaa atccccacca tctttcgaga 660
   tcccctgtaa cttattggca actgaaagaa tgaaaaggag gaaaatacaa aatatactag 720
   aactgaaaaa aaaaaagtat aaatagagac gatatatgcc aatacttcac aatgttcgaa 780
   tctattcttc atttgcagct attgtaaaat aataaaacat caagaacaaa caagctcaac 840
   ttgtcttttc taagaacaaa gaataaacac aaaaacaaaa agttttttta attttaatca 900
   aaaagttaac atgcatcacc atcaccatca cactagtgga tcccccgggc tgcaggaatt 960
   cgatatcaag cttatcgata ccgtcgacct cgagtcatgt aattagttat gtcacgctta 1020
   cattcacgcc ctccccccac atccgctcta accgaaaagg aaggagttag acaacctgaa 1080
   gtctaggtcc ctatttattt ttttatagtt atgttagtat taagaacgtt atttatattt 1140
   caaatttttc ttttttttct gtacagacgc gtgtacgcat gtaacattat actgaaaacc 1200
   ttgcttgaga aggttttggg acgctcgaag gctttaattt gcggccggta cccaattcgc 1260
   cctatagtga gtcgtattac gcgcgctcac tggccgtcgt tttacaacgt cgtgactggg 1320
   aaaaccctgg cgttacccaa cttaatcgcc ttgcagcaca tccccctttc gccagctggc 1380
   gtaatagcga agaggcccgc accgatcgcc cttcccaaca gttgcgcagc ctgaatggcg 1440
   aatggcgcga cgcgccctgt agcggcgcat taagcgcggc gggtgtggtg gttacgcgca 1500
   gcgtgaccgc tacacttgcc agcgccctag cgcccgctcc tttcgctttc ttcccttcct 1560
   ttctcgccac gttcgccggc tttccccgtc aagctctaaa tcgggggctc cctttagggt 1620
   tccgatttag tgctttacgg cacctcgacc ccaaaaaact tgattagggt gatggttcac 1680
   gtagtgggcc atcgccctga tagacggttt ttcgcccttt gacgttggag tccacgttct 1740
   ttaatagtgg actcttgttc caaactggaa caacactcaa ccctatctcg gtctattctt 1800
   ttgatttata agggattttg ccgatttcgg cctattggtt aaaaaatgag ctgatttaac 1860
   aaaaatttaa cgcgaatttt aacaaaatat taacgtttac aatttcctga tgcggtattt 1920
   tctccttacg catctgtgcg gtatttcaca ccgcataggg taataactga tataattaaa 1980
   ttgaagctct aatttgtgag tttagtatac atgcatttac ttataataca gttttttagt 2040
   tttgctggcc gcatcttctc aaatatgctt cccagcctgc ttttctgtaa cgttcaccct 2100
   ctaccttagc atcccttccc tttgcaaata gtcctcttcc aacaataata atgtcagatc 2160
   ctgtagagac cacatcatcc acggttctat actgttgacc caatgcgtct cccttgtcat 2220
   ctaaacccac accgggtgtc ataatcaacc aatcgtaacc ttcatctctt ccacccatgt 2280
   ctctttgagc aataaagccg ataacaaaat ctttgtcgct cttcgcaatg tcaacagtac 2340
   ccttagtata ttctccagta gatagggagc ccttgcatga caattctgct aacatcaaaa 2400
   ggcctctagg ttcctttgtt acttcttctg ccgcctgctt caaaccgcta acaatacctg 2460
   ggcccaccac accgtgtgca ttcgtaatgt ctgcccattc tgctattctg tatacacccg 2520
   cagagtactg caatttgact gtattaccaa tgtcagcaaa ttttctgtct tcgaagagta 2580
   aaaaattgta cttggcggat aatgccttta gcggcttaac tgtgccctcc atggaaaaat 2640
   cagtcaagat atccacatgt gtttttagta aacaaatttt gggacctaat gcttcaacta 2700
   actccagtaa ttccttggtg gtacgaacat ccaatgaagc acacaagttt gtttgctttt 2760
   cgtgcatgat attaaatagc ttggcagcaa caggactagg atgagtagca gcacgttcct 2820
   tatatgtagc tttcgacatg atttatcttc gtttcctgca ggtttttgtt ctgtgcagtt 2880
   gggttaagaa tactgggcaa tttcatgttt cttcaacact acatatgcgt atatatacca 2940
   atctaagtct gtgctccttc cttcgttctt ccttctgttc ggagattacc gaatcaaaaa 3000
   aatttcaaag aaaccgaaat caaaaaaaag aataaaaaaa aaatgatgaa ttgaattgaa 3060
   aagctgtggt atggtgcact ctcagtacaa tctgctctga tgccgcatag ttaagccagc 3120
   cccgacaccc gccaacaccc gctgacgcgc cctgacgggc ttgtctgctc ccggcatccg 3180
   cttacagaca agctgtgacc gtctccggga gctgcatgtg tcagaggttt tcaccgtcat 3240
   caccgaaacg cgcgagacga aagggcctcg tgatacgcct atttttatag gttaatgtca 3300
   tgataataat ggtttcttag tatgatccaa tatcaaagga aatgatagca ttgaaggatg 3360
   agactaatcc aattgaggag tggcagcata tagaacagct aaagggtagt gctgaaggaa 3420
   gcatacgata ccccgcatgg aatgggataa tatcacagga ggtactagac tacctttcat 3480
   cctacataaa tagacgcata taagtacgca tttaagcata aacacgcact atgccgttct 3540
   tctcatgtat atatatatac aggcaacacg cagatatagg tgcgacgtga acagtgagct 3600
   gtatgtgcgc agctcgcgtt gcattttcgg aagcgctcgt tttcggaaac gctttgaagt 3660
   tcctattccg aagttcctat tctctagaaa gtataggaac ttcagagcgc ttttgaaaac 3720
   caaaagcgct ctgaagacgc actttcaaaa aaccaaaaac gcaccggact gtaacgagct 3780
   actaaaatat tgcgaatacc gcttccacaa acattgctca aaagtatctc tttgctatat 3840
   atctctgtgc tatatcccta tataacctac ccatccacct ttcgctcctt gaacttgcat 3900
   ctaaactcga cctctacatt ttttatgttt atctctagta ttactcttta gacaaaaaaa 3960
   ttgtagtaag aactattcat agagtgaatc gaaaacaata cgaaaatgta aacatttcct 4020
   atacgtagta tatagagaca aaatagaaga aaccgttcat aattttctga ccaatgaaga 4080
   atcatcaacg ctatcacttt ctgttcacaa agtatgcgca atccacatcg gtatagaata 4140
   taatcgggga tgcctttatc ttgaaaaaat gcacccgcag cttcgctagt aatcagtaaa 4200
   cgcgggaagt ggagtcaggc tttttttatg gaagagaaaa tagacaccaa agtagccttc 4260
   ttctaacctt aacggaccta cagtgcaaaa agttatcaag agactgcatt atagagcgca 4320
   caaaggagaa aaaaagtaat ctaagatgct ttgttagaaa aatagcgctc tcgggatgca 4380
   tttttgtaga acaaaaaaga agtatagatt ctttgttggt aaaatagcgc tctcgcgttg 4440
   catttctgtt ctgtaaaaat gcagctcaga ttctttgttt gaaaaattag cgctctcgcg 4500
   ttgcattttt gttttacaaa aatgaagcac agattcttcg ttggtaaaat agcgctttcg 4560
   cgttgcattt ctgttctgta aaaatgcagc tcagattctt tgtttgaaaa attagcgctc 4620
   tcgcgttgca tttttgttct acaaaatgaa gcacagatgc ttcgttcagg tggcactttt 4680
   cggggaaatg tgcgcggaac ccctatttgt ttatttttct aaatacattc aaatatgtat 4740
   ccgctcatga gacaataacc ctgataaatg cttcaataat attgaaaaag gaagagtatg 4800
   agtattcaac atttccgtgt cgcccttatt cccttttttg cggcattttg ccttcctgtt 4860
   tttgctcacc cagaaacgct ggtgaaagta aaagatgctg aagatcagtt gggtgcacga     4920
   gtgggttaca tcgaactgga tctcaacagc ggtaagatcc ttgagagttt tcgccccgaa     4980
   gaacgttttc caatgatgag cacttttaaa gttctgctat gtggcgcggt attatcccgt     5040
   attgacgccg ggcaagagca actcggtcgc cgcatacact attctcagaa tgacttggtt     5100
   gagtactcac cagtcacaga aaagcatctt acggatggca tgacagtaag agaattatgc     5160
   agtgctgcca taaccatgag tgataacact gcggccaact tacttctgac aacgatcgga     5220
   ggaccgaagg agctaaccgc ttttttgcac aacatggggg atcatgtaac tcgccttgat     5280
   cgttgggaac cggagctgaa tgaagccata ccaaacgacg agcgtgacac cacgatgcct     5340
   gtagcaatgg caacaacgtt gcgcaaacta ttaactggcg aactacttac tctagcttcc     5400
   cggcaacaat taatagactg gatggaggcg gataaagttg caggaccact tctgcgctcg     5460
   gcccttccgg ctggctggtt tattgctgat aaatctggag ccggtgagcg tgggtctcgc     5520
   ggtatcattg cagcactggg gccagatggt aagccctccc gtatcgtagt tatctacacg     5580
   acggggagtc aggcaactat ggatgaacga aatagacaga tcgctgagat aggtgcctca     5640
   ctgattaagc attggtaact gtcagaccaa gtttactcat atatacttta gattgattta     5700
   aaacttcatt tttaatttaa aaggatctag gtgaagatcc tttttgataa tctcatgacc     5760
   aaaatccctt aacgtgagtt ttcgttccac tgagcgtcag accccgtaga aaagatcaaa     5820
   ggatcttctt gagatccttt ttttctgcgc gtaatctgct gcttgcaaac aaaaaaacca     5880
   ccgctaccag cggtggtttg tttgccggat caagagctac caactctttt tccgaaggta     5940
   actggcttca gcagagcgca gataccaaat actgtccttc tagtgtagcc gtagttaggc     6000
   caccacttca agaactctgt agcaccgcct acatacctcg ctctgctaat cctgttacca     6060
   gtggctgctg ccagtggcga taagtcgtgt cttaccgggt tggactcaag acgatagtta     6120
   ccggataagg cgcagcggtc gggctgaacg gggggttcgt gcacacagcc cagcttggag     6180
   cgaacgacct acaccgaact gagataccta cagcgtgagc tatgagaaag cgccacgctt     6240
   cccgaaggga gaaaggcgga caggtatccg gtaagcggca gggtcggaac aggagagcgc     6300
   acgagggagc ttccaggggg aaacgcctgg tatctttata gtcctgtcgg gtttcgccac     6360
<210> 7
   <211> 7590
   <212> DNA
   <213> Artificial Sequence
<220>
   <223> Vector
<400> 7
   ctctgacttg agcgtcgatt tttgtgatgc tcgtcagggg ggcggagcct atggaaaaac 60
   gccagcaacg cggccttttt acggttcctg gccttttgct ggccttttgc tcacatgttc 120
   tttcctgcgt tatcccctga ttctgtggat aaccgtatta ccgcctttga gtgagctgat 180
   accgctcgcc gcagccgaac gaccgagcgc agcgagtcag tgagcgagga agcggaagag 240
   cgcccaatac gcaaaccgcc tctccccgcg cgttggccga ttcattaatg cagctggcac 300
   gacaggtttc ccgactggaa agcgggcagt gagcgcaacg caattaatgt gagttacctc 360
   actcattagg caccccaggc tttacacttt atgcttccgg ctcctatgtt gtgtggaatt 420
   gtgagcggat aacaatttca cacaggaaac agctatgacc atgattacgc caagcgcgca 480
   attaaccctc actaaaggga acaaaagctg gagctcgtag gaacaatttc gggcccctgc 540
   gtgttcttct gaggttcatc ttttacattt gcttctgctg gataattttc agaggcaaca 600
   aggaaaaatt agatggcaaa aagtcgtctt tcaaggaaaa atccccacca tctttcgaga 660
   tcccctgtaa cttattggca actgaaagaa tgaaaaggag gaaaatacaa aatatactag 720
   aactgaaaaa aaaaaagtat aaatagagac gatatatgcc aatacttcac aatgttcgaa 780
   tctattcttc atttgcagct attgtaaaat aataaaacat caagaacaaa caagctcaac 840
   ttgtcttttc taagaacaaa gaataaacac aaaaacaaaa agttttttta attttaatca 900
   aaaaatggtg gctggaccaa acaaagatct tgaaaacctg gaacgtatga tgtactggaa 960
   gaccactttg aaagcttggt catgtttcct tgttggtgct aaattaaacg aaaaattaga 1020
   aacagatgat attttaaaag gtatccacaa attattcacg ttgagggttc agttacgttt 1080
   gaatgttttc caatatccta aaaaaaggtt tgttaccgaa gagataaatg gttggtctga 1140
   tgattttgtt gattttgtcg attatccaac tgatgatttt gatattattg aagcttttaa 1200
   acaacaacat aatcaatatt ttgaattggg tgttcaaaag cctttatgga aattggttgt 1260
   attgaaccat caatatttag ttattctttg tgatcatacc ttatatgatg ggaacactgc 1320
   actttatata tgtgaggatt tgatcacaat attgaatgat cgtgatatcc cagttgatag 1380
   aattccagat attaaaccat atcatgatct attaaaacca aaacttggac atacaatcaa 1440
   aactgtcatc caaacttttg caccaaaatg ggcttatcct ttagttaatc tgatttatag 1500
   accaaaaagt gaatttgaaa ctggtgcata tgatgattgg ggagtaactc ataaaattga 1560
   aagaacaaca aataaattaa agcacttaat tacaataact aatgaagaat tttccataat 1620
   taaaaaatta acaaaatcac atggtgtaaa tttcacagca ttttgggcat atatcaatgt 1680
   tcttgcagtt gcacaattgg gaaagtcagc tgttgattta tcaattccat tcaatatgag 1740
   aaccaattta ttaccaccag aatatttaag atggtatggt ttattagttt cacatgttac 1800
   tttaaatgta cataccaaag ttgatcatga ttcaattgac tgggattttg ttagattttt 1860
   aaatggtagt gttgcacata aataccaagt aaaacaatca caaatgcttg gaatgattaa 1920
   atatgttagt gctcgtggac ttattgaatc agctttaaaa tcaccaagaa aaggtggatt 1980
   agaagtttca aacttgggat tgagagtcga tccagatggt gaatcatgga aaaaatatac 2040
   ccctgaagaa tttttctttt ctttgccaaa tgatctttca ggttataatg tttcaaatgc 2100
   tgtgatttca agtaaaacta aaacaaatat tattttagac ggtgttccag aatttgcaaa 2160
   tgaatttcca acgtatgcaa ataacgttga aacaattttg agaaatgcaa tcaatgggta 2220
   ttatgaataa aattagttat gtcacgctta cattcacgcc ctccccccac atccgctcta 2280
   accgaaaagg aaggagttag acaacctgaa gtctaggtcc ctatttattt ttttatagtt 2340
   atgttagtat taagaacgtt atttatattt caaatttttc ttttttttct gtacagacgc 2400
   gtgtacgcat gtaacattat actgaaaacc ttgcttgaga aggttttggg acgctcgaag 2460
   gctttaattt gcggccggta cccaattcgc cctatagtga gtcgtattac gcgcgctcac 2520
   tggccgtcgt tttacaacgt cgtgactggg aaaaccctgg cgttacccaa cttaatcgcc 2580
   ttgcagcaca tccccctttc gccagctggc gtaatagcga agaggcccgc accgatcgcc 2640
   cttcccaaca gttgcgcagc ctgaatggcg aatggcgcga cgcgccctgt agcggcgcat 2700
   taagcgcggc gggtgtggtg gttacgcgca gcgtgaccgc tacacttgcc agcgccctag 2760
   cgcccgctcc tttcgctttc ttcccttcct ttctcgccac gttcgccggc tttccccgtc 2820
   aagctctaaa tcgggggctc cctttagggt tccgatttag tgctttacgg cacctcgacc 2880
   ccaaaaaact tgattagggt gatggttcac gtagtgggcc atcgccctga tagacggttt 2940
   ttcgcccttt gacgttggag tccacgttct ttaatagtgg actcttgttc caaactggaa 3000
   caacactcaa ccctatctcg gtctattctt ttgatttata agggattttg ccgatttcgg 3060
   cctattggtt aaaaaatgag ctgatttaac aaaaatttaa cgcgaatttt aacaaaatat 3120
   taacgtttac aatttcctga tgcggtattt tctccttacg catctgtgcg gtatttcaca 3180
   ccgcataggg taataactga tataattaaa ttgaagctct aatttgtgag tttagtatac 3240
   atgcatttac ttataataca gttttttagt tttgctggcc gcatcttctc aaatatgctt 3300
   cccagcctgc ttttctgtaa cgttcaccct ctaccttagc atcccttccc tttgcaaata 3360
   gtcctcttcc aacaataata atgtcagatc ctgtagagac cacatcatcc acggttctat 3420
   actgttgacc caatgcgtct cccttgtcat ctaaacccac accgggtgtc ataatcaacc 3480
   aatcgtaacc ttcatctctt ccacccatgt ctctttgagc aataaagccg ataacaaaat 3540
   ctttgtcgct cttcgcaatg tcaacagtac ccttagtata ttctccagta gatagggagc 3600
   ccttgcatga caattctgct aacatcaaaa ggcctctagg ttcctttgtt acttcttctg 3660
   ccgcctgctt caaaccgcta acaatacctg ggcccaccac accgtgtgca ttcgtaatgt 3720
   ctgcccattc tgctattctg tatacacccg cagagtactg caatttgact gtattaccaa 3780
   tgtcagcaaa ttttctgtct tcgaagagta aaaaattgta cttggcggat aatgccttta 3840
   gcggcttaac tgtgccctcc atggaaaaat cagtcaagat atccacatgt gtttttagta 3900
   aacaaatttt gggacctaat gcttcaacta actccagtaa ttccttggtg gtacgaacat 3960
   ccaatgaagc acacaagttt gtttgctttt cgtgcatgat attaaatagc ttggcagcaa 4020
   caggactagg atgagtagca gcacgttcct tatatgtagc tttcgacatg atttatcttc 4080
   gtttcctgca ggtttttgtt ctgtgcagtt gggttaagaa tactgggcaa tttcatgttt 4140
   cttcaacact acatatgcgt atatatacca atctaagtct gtgctccttc cttcgttctt 4200
   ccttctgttc ggagattacc gaatcaaaaa aatttcaaag aaaccgaaat caaaaaaaag 4260
   aataaaaaaa aaatgatgaa ttgaattgaa aagctgtggt atggtgcact ctcagtacaa 4320
   tctgctctga tgccgcatag ttaagccagc cccgacaccc gccaacaccc gctgacgcgc 4380
   cctgacgggc ttgtctgctc ccggcatccg cttacagaca agctgtgacc gtctccggga 4440
   gctgcatgtg tcagaggttt tcaccgtcat caccgaaacg cgcgagacga aagggcctcg 4500
   tgatacgcct atttttatag gttaatgtca tgataataat ggtttcttag tatgatccaa 4560
   tatcaaagga aatgatagca ttgaaggatg agactaatcc aattgaggag tggcagcata 4620
   tagaacagct aaagggtagt gctgaaggaa gcatacgata ccccgcatgg aatgggataa 4680
   tatcacagga ggtactagac tacctttcat cctacataaa tagacgcata taagtacgca 4740
   tttaagcata aacacgcact atgccgttct tctcatgtat atatatatac aggcaacacg 4800
   cagatatagg tgcgacgtga acagtgagct gtatgtgcgc agctcgcgtt gcattttcgg 4860
   aagcgctcgt tttcggaaac gctttgaagt tcctattccg aagttcctat tctctagaaa 4920
   gtataggaac ttcagagcgc ttttgaaaac caaaagcgct ctgaagacgc actttcaaaa 4980
   aaccaaaaac gcaccggact gtaacgagct actaaaatat tgcgaatacc gcttccacaa 5040
   acattgctca aaagtatctc tttgctatat atctctgtgc tatatcccta tataacctac 5100
   ccatccacct ttcgctcctt gaacttgcat ctaaactcga cctctacatt ttttatgttt 5160
   atctctagta ttactcttta gacaaaaaaa ttgtagtaag aactattcat agagtgaatc 5220
   gaaaacaata cgaaaatgta aacatttcct atacgtagta tatagagaca aaatagaaga 5280
   aaccgttcat aattttctga ccaatgaaga atcatcaacg ctatcacttt ctgttcacaa 5340
   agtatgcgca atccacatcg gtatagaata taatcgggga tgcctttatc ttgaaaaaat 5400
   gcacccgcag cttcgctagt aatcagtaaa cgcgggaagt ggagtcaggc tttttttatg 5460
   gaagagaaaa tagacaccaa agtagccttc ttctaacctt aacggaccta cagtgcaaaa 5520
   agttatcaag agactgcatt atagagcgca caaaggagaa aaaaagtaat ctaagatgct 5580
   ttgttagaaa aatagcgctc tcgggatgca tttttgtaga acaaaaaaga agtatagatt 5640
   ctttgttggt aaaatagcgc tctcgcgttg catttctgtt ctgtaaaaat gcagctcaga 5700
   ttctttgttt gaaaaattag cgctctcgcg ttgcattttt gttttacaaa aatgaagcac 5760
   agattcttcg ttggtaaaat agcgctttcg cgttgcattt ctgttctgta aaaatgcagc 5820
   tcagattctt tgtttgaaaa attagcgctc tcgcgttgca tttttgttct acaaaatgaa 5880
   gcacagatgc ttcgttcagg tggcactttt cggggaaatg tgcgcggaac ccctatttgt 5940
   ttatttttct aaatacattc aaatatgtat ccgctcatga gacaataacc ctgataaatg 6000
   cttcaataat attgaaaaag gaagagtatg agtattcaac atttccgtgt cgcccttatt 6060
   cccttttttg cggcattttg ccttcctgtt tttgctcacc cagaaacgct ggtgaaagta 6120
   aaagatgctg aagatcagtt gggtgcacga gtgggttaca tcgaactgga tctcaacagc 6180
   ggtaagatcc ttgagagttt tcgccccgaa gaacgttttc caatgatgag cacttttaaa 6240
   gttctgctat gtggcgcggt attatcccgt attgacgccg ggcaagagca actcggtcgc 6300
   cgcatacact attctcagaa tgacttggtt gagtactcac cagtcacaga aaagcatctt 6360
   acggatggca tgacagtaag agaattatgc agtgctgcca taaccatgag tgataacact 6420
   gcggccaact tacttctgac aacgatcgga ggaccgaagg agctaaccgc ttttttgcac 6480
   aacatggggg atcatgtaac tcgccttgat cgttgggaac cggagctgaa tgaagccata 6540
   ccaaacgacg agcgtgacac cacgatgcct gtagcaatgg caacaacgtt gcgcaaacta 6600
   ttaactggcg aactacttac tctagcttcc cggcaacaat taatagactg gatggaggcg 6660
   gataaagttg caggaccact tctgcgctcg gcccttccgg ctggctggtt tattgctgat 6720
   aaatctggag ccggtgagcg tgggtctcgc ggtatcattg cagcactggg gccagatggt 6780
   aagccctccc gtatcgtagt tatctacacg acggggagtc aggcaactat ggatgaacga 6840
   aatagacaga tcgctgagat aggtgcctca ctgattaagc attggtaact gtcagaccaa 6900
   gtttactcat atatacttta gattgattta aaacttcatt tttaatttaa aaggatctag 6960
   gtgaagatcc tttttgataa tctcatgacc aaaatccctt aacgtgagtt ttcgttccac 7020
   tgagcgtcag accccgtaga aaagatcaaa ggatcttctt gagatccttt ttttctgcgc 7080
   gtaatctgct gcttgcaaac aaaaaaacca ccgctaccag cggtggtttg tttgccggat 7140
   caagagctac caactctttt tccgaaggta actggcttca gcagagcgca gataccaaat 7200
   actgtccttc tagtgtagcc gtagttaggc caccacttca agaactctgt agcaccgcct 7260
   acatacctcg ctctgctaat cctgttacca gtggctgctg ccagtggcga taagtcgtgt 7320
   cttaccgggt tggactcaag acgatagtta ccggataagg cgcagcggtc gggctgaacg 7380
   gggggttcgt gcacacagcc cagcttggag cgaacgacct acaccgaact gagataccta 7440
   cagcgtgagc tatgagaaag cgccacgctt cccgaaggga gaaaggcgga caggtatccg 7500
   gtaagcggca gggtcggaac aggagagcgc acgagggagc ttccaggggg aaacgcctgg 7560
   tatctttata gtcctgtcgg gtttcgccac 7590
<210> 8
   <211> 8933
   <212> DNA
   <213> Artificial Sequence
<220>
   <223> Vector
<400> 8
   cgtcaggggg gcggagccta tggaaaaacg ccagcaacgc ggccttttta cggttcctgg 60
   ccttttgctg gccttttgct cacatgttct ttcctgcgtt atcccctgat tctgtggata 120
   accgtattac cgcctttgag tgagctgata ccgctcgccg cagccgaacg accgagcgca 180
   gcgagtcagt gagcgaggaa gcggaagagc gcccaatacg caaaccgcct ctccccgcgc 240
   gttggccgat tcattaatgc agctggcacg acaggtttcc cgactggaaa gcgggcagtg 300
   agcgcaacgc aattaatgtg agttacctca ctcattaggc accccaggct ttacacttta 360
   tgcttccggc tcctatgttg tgtggaattg tgagcggata acaatttcac acaggaaaca 420
   gctatgacca tgattacgcc aagcgcgcaa ttaaccctca ctaaagggaa caaaagctgg 480
   agctcgtagg aacaatttcg ggcccctgcg tgttcttctg aggttcatct tttacatttg 540
   cttctgctgg ataattttca gaggcaacaa ggaaaaatta gatggcaaaa agtcgtcttt 600
   caaggaaaaa tccccaccat ctttcgagat cccctgtaac ttattggcaa ctgaaagaat 660
   gaaaaggagg aaaatacaaa atatactaga actgaaaaaa aaaaagtata aatagagacg 720
   atatatgcca atacttcaca atgttcgaat ctattcttca tttgcagcta ttgtaaaata 780
   ataaaacatc aagaacaaac aagctcaact tgtcttttct aagaacaaag aataaacaca 840
   aaaacaaaaa gtttttttaa ttttaatcaa aaagtgaata tattttttca agggcccata 900
   tatatatata aacacaagaa atgtttattt gaatcattac aatttacttt cttaacctca 960
   tcatcaaata tgtcatttaa atatatcaat caaaatgatt caaaatcatt atcaaattta 1020
   aaatataaat tatcaaaaaa tcatgcaaga caaatgggtt ttttagaaga tttttttgca 1080
   attttacaac gtcaaaaaat gtataaatca tttttcgtta tgtgtaaata taatgaaaaa 1140
   attgatgatt ttaaaatttt attccattca ttaagattat taatattaaa attcccaata 1200
   ttagcttcca caataattac tcaaaatgtt ccaattaata taaaacctcg tccttatgat 1260
   tatattcaaa ttattgatga aataaaattt aatgatttgg tttgggattt aagacctgaa 1320
   tattcaaatt tattacaaga agatttatta aataaattaa atgatttaat tataccatat 1380
   gaagataata aattagtttg gagattagga atcttggatg attatacatt aatttttata 1440
   acaaatcatg ttttacatga tggaatatct ggtaaaaata tttttaatga attatcatta 1500
   atttttaatc aattggactt ggattcttta agtgatgatg atgatatcgt gttcaattat 1560
   tcacaagatc atttgaattt aggtgaatta ccaaaaccta taactgatct tatgaatcat 1620
   attccatcaa ttaaatcttt accaagatat atttataatt cattaattga accaaaactt 1680
   ttttgttcat caactttaat tcaaggtcat cttaagaata ttcattatag agttaatata 1740
   aatccaatgg aattattaaa aattaaatca ttattatcaa aaaatagttt caataatgtt 1800
   aaattaactt taacaccttt cattcaatct atttggaatt atactttata tcaagatgaa 1860
   tattataaat catcaaaatc tttattaggt attgcagtgg attctcgtca atttattaat 1920
   aaagatgaac aagatttata taaatttggt ttaaatgtat caggttttag taaaatttcc 1980
   aaaccaatga aattaattac atggaataaa attaatcaaa ttaatcaaga tttaaaaatt 2040
   tcattaaaat tgaaaaaacc tttatattca atgggtatat taggttggga taaaatgatt 2100
   aaaaataaac atttagatgt tgatttacca aaaattatga ataaaagaac aggttcaact 2160
   ttttcaaata ttggtataat cctaaataac agtgaatcaa atgataaatt tcaaattatt 2220
   gatgcaatgt ttacacaaca ttttaatgtt catttttatg atttctcaat cactgcaatt 2280
   tctacaatga ctggtgggtt aaatattata attacatcac cagaatctat tggaattgaa 2340
   aatttagaaa gaatttgtaa aaaatttcat gaaaatttag ttttatgtga tattaaataa 2400
   ggataatttg ggcccgatga attagttatg tcacgcttac attcacgccc tccccccaca 2460
   tccgctctaa ccgaaaagga aggagttaga caacctgaag tctaggtccc tatttatttt 2520
   tttatagtta tgttagtatt aagaacgtta tttatatttc aaatttttct tttttttctg 2580
   tacagacgcg tgtacgcatg taacattata ctgaaaacct tgcttgagaa ggttttggga 2640
   cgctcgaagg ctttaatttg cggccggtac ccaattcgcc ctatagtgag tcgtattacg 2700
   cgcgctcact ggccgtcgtt ttacaacgtc gtgactggga aaaccctggc gttacccaac 2760
   ttaatcgcct tgcagcacat ccccctttcg ccagctggcg taatagcgaa gaggcccgca 2820
   ccgatcgccc ttcccaacag ttgcgcagcc tgaatggcga atggcgcgac gcgccctgta 2880
   gcggcgcatt aagcgcggcg ggtgtggtgg ttacgcgcag cgtgaccgct acacttgcca 2940
   gcgccctagc gcccgctcct ttcgctttct tcccttcctt tctcgccacg ttcgccggct 3000
   ttccccgtca agctctaaat cgggggctcc ctttagggtt ccgatttagt gctttacggc 3060
   acctcgaccc caaaaaactt gattagggtg atggttcacg tagtgggcca tcgccctgat 3120
   agacggtttt tcgccctttg acgttggagt ccacgttctt taatagtgga ctcttgttcc 3180
   aaactggaac aacactcaac cctatctcgg tctattcttt tgatttataa gggattttgc 3240
   cgatttcggc ctattggtta aaaaatgagc tgatttaaca aaaatttaac gcgaatttta 3300
   acaaaatatt aacgtttaca atttcctgat gcggtatttt ctccttacgc atctgtgcgg 3360
   tatttcacac cgcatatcga cggtcgagga gaacttctag tatatccaca tacctaatat 3420
   tattgcctta ttaaaaatgg aatcccaaca attacatcaa aatccacatt ctcttcaaaa 3480
   tcaattgtcc tgtacttcct tgttcatgtg tgttcaaaaa cgttatattt ataggataat 3540
   tatactctat ttctcaacaa gtaattggtt gtttggccga gcggtctaag gcgcctgatt 3600
   caagaaatat cttgaccgca gttaactgtg ggaatactca ggtatcgtaa gatgcaagag 3660
   ttcgaatctc ttagcaacca ttattttttt cctcaacata acgagaacac acaggggcgc 3720
   tatcgcacag aatcaaattc gatgactgga aattttttgt taatttcaga ggtcgcctga 3780
   cgcatatacc tttttcaact gaaaaattgg gagaaaaagg aaaggtgaga ggccggaacc 3840
   ggcttttcat atagaataga gaagcgttca tgactaaatg cttgcatcac aatacttgaa 3900
   gttgacaata ttatttaagg acctattgtt ttttccaata ggtggttagc aatcgtctta 3960
   ctttctaact tttcttacct tttacatttc agcaatatat atatatattt caaggatata 4020
   ccattctaat gtctgcccct atgtctgccc ctaagaagat cgtcgttttg ccaggtgacc 4080
   acgttggtca agaaatcaca gccgaagcca ttaaggttct taaagctatt tctgatgttc 4140
   gttccaatgt caagttcgat ttcgaaaatc atttaattgg tggtgctgct atcgatgcta 4200
   caggtgtccc acttccagat gaggcgctgg aagcctccaa gaaggttgat gccgttttgt 4260
   taggtgctgt ggctggtcct aaatggggta ccggtagtgt tagacctgaa caaggtttac 4320
   taaaaatccg taaagaactt caattgtacg ccaacttaag accatgtaac tttgcatccg 4380
   actctctttt agacttatct ccaatcaagc cacaatttgc taaaggtact gacttcgttg 4440
   ttgtcagaga attagtggga ggtatttact ttggtaagag aaaggaagac gatggtgatg 4500
   gtgtcgcttg ggatagtgaa caatacaccg ttccagaagt gcaaagaatc acaagaatgg 4560
   ccgctttcat ggccctacaa catgagccac cattgcctat ttggtccttg gataaagcta 4620
   atcttttggc ctcttcaaga ttatggagaa aaactgtgga ggaaaccatc aagaacgaat 4680
   tccctacatt gaaggttcaa catcaattga ttgattctgc cgccatgatc ctagttaaga 4740
   acccaaccca cctaaatggt attataatca ccagcaacat gtttggtgat atcatctccg 4800
   atgaagcctc cgttatccca ggttccttgg gtttgttgcc atctgcgtcc ttggcctctt 4860
   tgccagacaa gaacaccgca tttggtttgt acgaaccatg ccacggttct gctccagatt 4920
   tgccaaagaa taaggttgac cctatcgcca ctatcttgtc tgctgcaatg atgttgaaat 4980
   tgtcattgaa cttgcctgaa gaaggtaagg ccattgaaga tgcagttaaa aaggttttgg 5040
   atgcaggtat cagaactggt gatttaggtg gttccaacag taccaccgaa gtcggtgatg 5100
   ctgtcgccga agaagttaag aaaatccttg cttaaaaaga ttctcttttt ttatgatatt 5160
   tgtacataaa ctttataaat gaaattcata atagaaacga cacgaaatta caaaatggaa 5220
   tatgttcata gggtagacga aactatatac gcaatctaca tacatttatc aagaaggaga 5280
   aaaaggagga tagtaaagga atacaggtaa gcaaattgat actaatggct caacgtgata 5340
   aggaaaaaga attgcacttt aacattaata ttgacaagga ggagggcacc acacaaaaag 5400
   ttaggtgtaa cagaaaatca tgaaactacg attcctaatt tgatattgga ggattttctc 5460
   taaaaaaaaa aaaatacaac aaataaaaaa cactcaatga cctgaccatt tgatggagtt 5520
   taagtcaata ccttcttgaa gcatttccca taatggtgaa agttccctca agaattttac 5580
   tctgtcagaa acggccttac gacgtagtcg atatggtgca ctctcagtac aatctgctct 5640
   gatgccgcat agttaagcca gccccgacac ccgccaacac ccgctgacgc gccctgacgg 5700
   gcttgtctgc tcccggcatc cgcttacaga caagctgtga ccgtctccgg gagctgcatg 5760
   tgtcagaggt tttcaccgtc atcaccgaaa cgcgcgagac gaaagggcct cgtgatacgc 5820
   ctatttttat aggttaatgt catgataata atggtttctt agtatgatcc aatatcaaag 5880
   gaaatgatag cattgaagga tgagactaat ccaattgagg agtggcagca tatagaacag 5940
   ctaaagggta gtgctgaagg aagcatacga taccccgcat ggaatgggat aatatcacag 6000
   gaggtactag actacctttc atcctacata aatagacgca tataagtacg catttaagca 6060
   taaacacgca ctatgccgtt cttctcatgt atatatatat acaggcaaca cgcagatata 6120
   ggtgcgacgt gaacagtgag ctgtatgtgc gcagctcgcg ttgcattttc ggaagcgctc 6180
   gttttcggaa acgctttgaa gttcctattc cgaagttcct attctctaga aagtatagga 6240
   acttcagagc gcttttgaaa accaaaagcg ctctgaagac gcactttcaa aaaaccaaaa 6300
   acgcaccgga ctgtaacgag ctactaaaat attgcgaata ccgcttccac aaacattgct 6360
   caaaagtatc tctttgctat atatctctgt gctatatccc tatataacct acccatccac 6420
   ctttcgctcc ttgaacttgc atctaaactc gacctctaca ttttttatgt ttatctctag 6480
   tattactctt tagacaaaaa aattgtagta agaactattc atagagtgaa tcgaaaacaa 6540
   tacgaaaatg taaacatttc ctatacgtag tatatagaga caaaatagaa gaaaccgttc 6600
   ataattttct gaccaatgaa gaatcatcaa cgctatcact ttctgttcac aaagtatgcg 6660
   caatccacat cggtatagaa tataatcggg gatgccttta tcttgaaaaa atgcacccgc 6720
   agcttcgcta gtaatcagta aacgcgggaa gtggagtcag gcttttttta tggaagagaa 6780
   aatagacacc aaagtagcct tcttctaacc ttaacggacc tacagtgcaa aaagttatca 6840
   agagactgca ttatagagcg cacaaaggag aaaaaaagta atctaagatg ctttgttaga 6900
   aaaatagcgc tctcgggatg catttttgta gaacaaaaaa gaagtataga ttctttgttg 6960
   gtaaaatagc gctctcgcgt tgcatttctg ttctgtaaaa atgcagctca gattctttgt 7020
   ttgaaaaatt agcgctctcg cgttgcattt ttgttttaca aaaatgaagc acagattctt 7080
   cgttggtaaa atagcgcttt cgcgttgcat ttctgttctg taaaaatgca gctcagattc 7140
   tttgtttgaa aaattagcgc tctcgcgttg catttttgtt ctacaaaatg aagcacagat 7200
   gcttcgttca ggtggcactt ttcggggaaa tgtgcgcgga acccctattt gtttattttt 7260
   ctaaatacat tcaaatatgt atccgctcat gagacaataa ccctgataaa tgcttcaata 7320
   atattgaaaa aggaagagta tgagtattca acatttccgt gtcgccctta ttcccttttt      7380
   tgcggcattt tgccttcctg tttttgctca cccagaaacg ctggtgaaag taaaagatgc      7440
   tgaagatcag ttgggtgcac gagtgggtta catcgaactg gatctcaaca gcggtaagat      7500
   ccttgagagt tttcgccccg aagaacgttt tccaatgatg agcactttta aagttctgct      7560
   atgtggcgcg gtattatccc gtattgacgc cgggcaagag caactcggtc gccgcataca      7620
   ctattctcag aatgacttgg ttgagtactc accagtcaca gaaaagcatc ttacggatgg      7680
   catgacagta agagaattat gcagtgctgc cataaccatg agtgataaca ctgcggccaa      7740
   cttacttctg acaacgatcg gaggaccgaa ggagctaacc gcttttttgc acaacatggg      7800
   ggatcatgta actcgccttg atcgttggga accggagctg aatgaagcca taccaaacga      7860
   cgagcgtgac accacgatgc ctgtagcaat ggcaacaacg ttgcgcaaac tattaactgg      7920
   cgaactactt actctagctt cccggcaaca attaatagac tggatggagg cggataaagt      7980
   tgcaggacca cttctgcgct cggcccttcc ggctggctgg tttattgctg ataaatctgg      8040
   agccggtgag cgtgggtctc gcggtatcat tgcagcactg gggccagatg gtaagccctc      8100
   ccgtatcgta gttatctaca cgacggggag tcaggcaact atggatgaac gaaatagaca      8160
   gatcgctgag ataggtgcct cactgattaa gcattggtaa ctgtcagacc aagtttactc      8220
   atatatactt tagattgatt taaaacttca tttttaattt aaaaggatct aggtgaagat      8280
   cctttttgat aatctcatga ccaaaatccc ttaacgtgag ttttcgttcc actgagcgtc      8340
   agaccccgta gaaaagatca aaggatcttc ttgagatcct ttttttctgc gcgtaatctg      8400
   ctgcttgcaa acaaaaaaac caccgctacc agcggtggtt tgtttgccgg atcaagagct      8460
   accaactctt tttccgaagg taactggctt cagcagagcg cagataccaa atactgtcct      8520
   tctagtgtag ccgtagttag gccaccactt caagaactct gtagcaccgc ctacatacct      8580
   cgctctgcta atcctgttac cagtggctgc tgccagtggc gataagtcgt gtcttaccgg      8640
   gttggactca agacgatagt taccggataa ggcgcagcgg tcgggctgaa cggggggttc      8700
   gtgcacacag cccagcttgg agcgaacgac ctacaccgaa ctgagatacc tacagcgtga      8760
   gctatgagaa agcgccacgc ttcccgaagg gagaaaggcg gacaggtatc cggtaagcgg      8820
   cagggtcgga acaggagagc gcacgaggga gcttccaggg ggaaacgcct ggtatcttta      8880
   tagtcctgtc gggtttcgcc acctctgact tgagcgtcga tttttgtgat gct             8933
<210> 9
   <211> 57
   <212> DNA
   <213> Artificial Sequence
<220>
   <223> Primer
<400> 9
   aaaacaaaaa gtttttttaa ttttaatcaa aaagtgaata tattttttca agggccc 57
<210> 10
   <211> 52
   <212> DNA
   <213> Artificial Sequence
<220>
   <223> Primer
<400> 10
   gagggcgtga atgtaagcgt gacataacta attcatcggg cccaaattat cc 52
<210> 11
   <211> 51
   <212> DNA
   <213> Artificial Sequence
<220>
   <223> Primer
<400> 11
   aaaacaaaaa gtttttttaa ttttaatcaa aaaatggtgg ctggaccaaa c 51
<210> 12
   <211> 59
   <212> DNA
   <213> Artificial Sequence
<220>
   <223> Primer
<400> 12
   gggagggcgt gaatgtaagc gtgacataac taattttatt cataataccc attgattgc 59